# EUROPEAN PATENT APPLICATION

(11) **EP 4 295 860 A1**
(43) Date of publication of application: **27.12.2023**
(21) Application number: 22180820.7
(22) Date of filing: 23.06.2022
(51) Int. Cl.: A61K 39/00, C07K 16/28, C07K 14/725

(54) **ENGINEERED HUMAN T CELLS COMPRISING A SWITCHABLE CHIMERIC ANTIGEN CELL SURFACE RECEPTOR AND METHODS FOR GENERATING THEM**

(71) Applicant: Ehninger, Armin, 01307 Dresden (DE); Cartellieri, Marc, 01307 Dresden (DE); Lescarbeau, Reynald, Cambridge, MA 02138 (US); Ehninger, Gerhard, 01307 Dresden (DE); Schiermeier, Andrew, Cambridge, MA 02138 (US)
(72) Inventor: Ehninger, Armin, 01307 Dresden (DE); Cartellieri, Marc, 01307 Dresden (DE); Lescarbeau, Reynald, Cambridge, MA 02138 (US); Ehninger, Gerhard, 01307 Dresden (DE); Schiermeier, Andrew, Cambridge, MA 02138 (US)
(74) Representative: Kailuweit & Uhlemann Patentanwälte Partnerschaft mbB

(57) **Abstract**

The present invention relates to an engineered human T cell comprising a switchable chimeric antigen cell surface receptor, a pharmaceutical composition comprising the engineered human T cell, a kit comprising the engineered human T cell and a targeting module and a method for generating the engineered human T cell.

## Description

The present invention relates to an engineered human T cell comprising a switchable chimeric antigen cell surface receptor, a pharmaceutical composition comprising the engineered human T cell, a kit comprising the engineered human T cell and a targeting module and a method for generating the engineered human T cell.

Chimeric antigen receptors (CARs) are artificial receptors consisting of a binding moiety, which provides the antigen-specificity and one or several signaling chains derived from immune receptors (Cartellieri *et al.* 2010). Immune cells, genetically modified to express CARs, can be used to bind cells or tissue structures expressing the appropriate target of the CAR binding moiety. Cross-linking leads to an induction of signal pathways via the CAR signaling chains, which will change the biologic properties of the CAR-engrafted immune cell. In contrast, CAR activation in gene-modified regulatory T cells (Tregs) leads to an activation of Treg-specific immunomodulatory and suppressive mechanisms like interleukin (IL)-10 or tumor growth factor-beta (TGF-β) secretion. The adoptive transfer of immune cells engineered with chimeric antigen receptors (CARs) is currently considered a highly promising therapeutic option for the treatment of otherwise incurable malignant, infectious or autoimmune diseases.

However, the conventional CAR technology comes along with a number of critical issues, which need to be solved before this treatment modality can be widely applied for clinical treatments. First of all, several safety issues have to be addressed. So far, immune responses of T cells engineered with conventional CARs are difficult to control after infusion into the patient. Serious adverse event rates are high (Titov *et al.* 2018). Especially unexpected target gene expression on normal tissue may provoke a rapid and rigorous immune reaction of engineered T cells against normal cells, which can cause severe side effects (Morgan *et al.* 2010). Moreover, as CAR-T cells are a new class of self-amplifying cell drugs, infused T cells can undergo a vigorous expansion in the presence of heavy tumor burden leading to tumor lysis syndrome, cytokine release syndrome and macrophage activation syndrome (Brudno and Kochenderfer 2016). Another drawback of conventional CAR technology is the restriction of engineered T cell retargeting to a single antigen. Such a monotherapeutic approach implies the risk for the development of tumor escape variants, which have lost the target antigen during treatment. The emergence of tumor escape variants under conventional CAR T cell therapy after several months was already observed in clinical trials (Sotillo *et al.* 2015). Taken together, these obstacles restrict the application of CAR T cells to very few indications. In fact, examples of clinical effectiveness have mostly been seen with CD19- and BCMA-targeting CAR T cells until now.

Modular switchable "universal" CAR T (UniCAR) approaches can overcome these limitations by separating antigen recognition and activating domain of a CAR into two separate operational units. T cells are engineered to express a CAR with a universal binding domain recognizing atag (Cartellieri *et al.* 2016). Antigen-specificity is provided by soluble adapter molecules, which consist of an antigen-binding domain fused to the tag recognized by the universal CAR. Cartellieri *et al.* describe the treatment of CD33- and/or CD123-positive acute myeloid leukemia cells *in vitro* and *in vivo.*

Next to the UniCAR approach for recognizing various antigens (EP 2 990 416 A1) a reversed universal CAR (RevCAR) approach is known that promotes binding of an immune cell engineered to express a RevCAR comprising a tag to a target cell through an adaptor molecule comprising a tag-binding domain and a target cell binding domain (EP 3 581 200 A1).

Moreover, switchable CAR-T approaches like UniCAR or RevCAR provide the possibility to rest CAR T cells in-between cycles of activation and stimulation by pausing administration of the soluble adapter molecule. This is expected to prevent the exhaustion observed upon continuous stimulation of conventional CAR T cells thereby improving persistence (Weber et al. 2021).

To avoid problems of immune rejection, for engineering immune cells with a UniCAR or RevCAR autologous approaches are applied that use subject's own cells as the cell source for therapy. This approach however is time-consuming and costly, and the patients have usually undergone several lines of prior treatment, which may have adversely affected immune cell functional properties.

To overcome this problem, the transfer of allogeneic cells into a subject could be applied. However, the use of allogeneic cells has been limited due to the problem of rejection by the recipient subject's immune cells, which recognize the transplanted cells as foreign and mount an attack. Immune rejection of allogeneic cells normally results from mismatching of major histocompatibility complex (MHC) molecules between donor and recipient. MHC molecules exist within the human population in numerous genetic variants of any given MHC gene, encoding different forms of MHC protein. MHC class I molecules comprise HLA-A, HLA-B, and HLA-C molecules in humans, which are expressed on all nucleated cells and also in platelets and present epitopes to activate cytotoxic T cells. MHC class II molecules comprise HLA-DP, HLA-DQ and HLA-DR molecules and are expressed on only specified antigen-presenting cells, e. g. macrophages, B cells and dendritic cells (DCs), and present antigens to activate helper T cells, which in turn provide signals to B cells to produce antibodies.

Slight differences in MHC alleles between individuals can cause the T cells in a recipient to become activated upon transfer of donor T cells. During T cell development, an individual's T cell repertoire is tolerized to one's own MHC molecules, but T cellsthat recognize another individual's MHC molecules may persist in circulation and are referred to as alloreactive T cells. Alloreactive T cells can become activated by the presence of another individual's cells expressing MHC molecules in the body, causing graft versus host disease and transplant rejection.

Methods and compositions for reducing the susceptibility of an allogeneic cell to rejection are of interest, including reducing the cell's expression of MHC protein to avoid recipient T cell responses. In practice, the ability to genetically modify an allogeneic cell for transplantation into a subject has been hampered by the requirement for multiple gene edits to reduce all MHC protein expression. While at the same time, avoiding other harmful recipient immune responses. One approach is to utilize prolonged immune suppression to avoid immune rejection and increase persistence (Neelapu *et al.* 2020). Although showing responses in the clinic, this approach increases the risk of infections and the durability of the adoptive T cells is uncertain and depends on ongoing immune suppression. Other strategies include knock out of the β2M gene to remove all HLA class I molecules (HLA-A, HLA-B and HLA-C) from the T cell surface, to avoid recognition by host CD8+ T cells. However, loss of HLA class I sends a "missing-self" signal to host NK cells, which readily eliminate β2M KO T cells. To overcome this, researchers are exploring overexpression of the non-polymorphic HLA-E gene, which can provide partial but not full protection from NK cell-mediated lysis (Zhang 2021).

The object of the present invention is therefore to provide an engineered allogeneic immune cell comprising a switchable chimeric antigen cell surface receptor, which causes reduced rejection by the recipient subject's immune cells and has preferably superior persistence by combining avoidance of rejection with avoidance of continuous stimulation resulting in exhaustion.

According to the invention, the task is solved by the engineered human T cell, the kit and the method for generating an engineered human T cell according to the independent claims.

Advantageous embodiments of the invention are indicated in the dependent claims.

A first aspect of the invention provides an engineered human T cell, comprising:
i. reduced or eliminated surface expression of endogenous T cell receptor alpha chain (TRAC) by a genetic modification in the T cell receptoralpha chain gene,
ii. reduced or eliminated surface expression of HLA-A relative to an unmodified T cell by a genetic modification in the HLA-A gene,
iii. reduced or eliminated surface expression of HLA class II by a genetic modification in the CIITA gene, and
iv. a switchable chimeric antigen cell surface receptor that comprises:
   - a tag-binding domain or a tag,
   - an extracellular hinge and a transmembrane domain, and
   - a signal transduction domain.

Advantageously, the engineered human T cell according to the invention used in combination with a targeting module actively targeting an antigen expressed by tumor cells and is capable of inducing a significant anti-tumor response, wherein the anti-tumor response of the switchable chimeric antigen receptor is only been induced in the presence of the targeting module. The effect can be reversibly interrupted by withholding the administration of the targeting module. Further advantageously, the pharmacokinetic and pharmacodynamic half-life of the targeting module is short, providing a rapid and reversible switch-off mechanism of the mediated immune response.

As used herein, the term "domain" refers to a part of a protein sequence, which can exist and function independently from the rest of the protein.

According to the invention, the engineered human T cell comprises reduced or eliminated surface expression of endogenous T cell receptor alpha chain (TRAC) by a genetic modification in the TRAC gene. Preferably, the reduced or eliminated surface expression of endogenous TRAC results in to preventing graft-versus-host disease.

In embodiments, the genetic modification in the TRAC gene comprises at least one nucleotide within the genomic coordinates chr14:22547524-chr14:22547544. In embodiments, the genetic modification in the TRAC gene comprises at least 10 or at least 15 contiguous nucleotides within the genomic coordinates.

In embodiments, the genetic modification in the TRAC gene comprises at least one nucleotide of an exon of the TRAC gene.

In embodiments, the genetic modification in the TRAC gene comprises at least one insertion, deletion, substitution, or deamination of at least one nucleotide within the genomic coordinates.

In embodiments, the genetic modification in the TRAC gene comprises an indel.

In embodiments, the TRAC gene expression is reduced or eliminated by a gene editing system that binds to a TRAC genomic target sequence comprising at least 5 contiguous nucleotides within the genomic coordinates, preferably within the genomic coordinates chr14:22547524-chr14:22547544.

According to the invention, the engineered human T cell comprises a reduced or eliminated surface expression of HLA-A relative to an unmodified T cell by a genetic modification in the HLA-A gene.

In embodiments, the genetic modification in the HLA-A gene comprises at least one nucleotide within the genomic coordinates chosen from: chr6:29942854-chr6:29942913 and chr6:29943518-chr6:29943619.

In embodiments, the T cell is homozygous for HLA-B and/or homozygous for HLA-C genotypes. Preferably, the T cell is homozygous for HLA-B and HLA-C genotypes.

In embodiments, the T cell has reduced or eliminated expression of at least one HLA-A allele selected from: HLA-A1, HLA-A2, HLA-A3, HLA-A11, and HLA-A24.

In embodiments, the genetic modification in the HLA-A gene comprises at least one nucleotide within the genomic coordinates chr6:29942864-chr6:29942903, preferably chr6:29942876-chr6:29942897.

In embodiments, the genetic modification in the HLA-A gene comprises at least one nucleotide within the genomic coordinates chr6:29943528-chr6:29943609, preferably chr6:29943528-chr6:29943550.

In embodiments, the genetic modification in the HLA-A gene comprises at least 5, 6, 7, 8, 9, or 10 contiguous nucleotides within the genomic coordinates, preferably at least 10, at least 15, at least 16, at least 17, at least 18, at least 19, or at least 20 contiguous nucleotides within the genomic coordinates.

In embodiments, the genetic modification in the HLA-A gene comprises at least one C to T substitution or at least one A to G substitution within the genomic coordinates.

In embodiments, the genetic modification in the HLA-A gene comprises an indel.

In embodiments, the HLA-A expression is reduced or eliminated by a gene editing system that binds to an HLA-A genomic target sequence comprising at least 5 contiguous nucleotides within the genomic coordinates, preferably within the genomic coordinates chosen from: chr6:29942864-29942884; chr6:29942868-29942888; chr6:29942876-29942896; chr6:29942877-29942897; chr6:29942883-29942903; chr6:29943126-29943146; chr6:29943528-29943548; chr6:29943529-29943549; chr6:29943530-29943550; chr6:29943537-29943557; chr6:29943549-29943569; chr6:29943589-29943609; and chr6:29944026-29944046.

In embodiments, HLA-B allele is selected from any one of the following HLA-B alleles: HLA-B*07:02; HLA-B*08:01; HLA-B*44:02; HLA-B*35:01; HLA-B*40:01; HLA-B*57:01; HLA-B*14:02; HLA-B*15:01; HLA-B*13:02; HLA-B*44:03; HLA-B*38:01; HLA-B*18:01; HLA-B*44:03; HLA-B*51:01; HLA-B*49:01; HLA-B*15:01; HLA-B*18:01; HLA-B*27:05; HLA-B*35:03; HLA-B*18:01; HLA-B*52:01; HLA-B*51:01; HLA-B*37:01; HLA-B*53:01; HLA-B*55:01; HLA-B*44:02; HLA-B*44:03; HLA-B*35:02; HLA-B*15:01; and HLA-B*40:02.

In embodiments, the HLA-C allele is selected from any one of the following HLA-C alleles: HLA-C*07:02; HLA-C*07:01; HLA-C*05:01; HLA-C*04:01 HLA-C*03:04; HLA-C*06:02; HLA-C*08:02; HLA-C*03:03; HLA-C*06:02; HLA-C*16:01; HLA-C*12:03; HLA-C*07:01; HLA-C*04:01; HLA-C*15:02; HLA-C*07:01; HLA-C*03:04; HLA-C*12:03; HLA-C*02:02; HLA-C*04:01; HLA-C*05:01; HLA-C*12:02; HLA-C*14:02; HLA-C*06:02; HLA-C*04:01; HLA-C*03:03; HLA-C*07:04; HLA-C*07:01; HLA-C*04:01; HLA-C*04:01; and HLA-C*02:02.

In embodiments, HLA-B allele is selected from any one of the following HLA-B alleles: HLA-B*07:02; HLA-B*08:01; HLA-B*44:02; HLA-B*35:01; HLA-B*40:01; HLA-B*57:01; HLA-B*14:02; HLA-B*15:01; HLA-B*13:02; HLA-B*44:03; HLA-B*38:01; HLA-B*18:01; HLA-B*44:03; HLA-B*51:01; HLA-B*49:01; HLA-B*15:01; HLA-B*18:01; HLA-B*27:05; HLA-B*35:03; HLA-B*18:01; HLA-B*52:01; HLA-B*51:01; HLA-B*37:01; HLA-B*53:01; HLA-B*55:01; HLA-B*44:02; HLA-B*44:03; HLA-B*35:02; HLA-B*15:01; and HLA-B*40:02; and the HLA-C allele is selected from anyone of the following HLA-C alleles: HLA-C*07:02; HLA-C*07:01; HLA-C*05:01; HLA-C*04:01 HLA-C*03:04; HLA-C*06:02; HLA-C*08:02; HLA-C*03:03; HLA-C*06:02; HLA-C*16:01; HLA-C*12:03; HLA-C*07:01; HLA-C*04:01; HLA-C*15:02; HLA-C*07:01; HLA-C*03:04; HLA-C*12:03; HLA-C*02:02; HLA-C*04:01; HLA-C*05:01; HLA-C*12:02; HLA-C*14:02; HLA-C*06:02; HLA-C*04:01; HLA-C*03:03; HLA-C*07:04; HLA-C*07:01; HLA-C*04:01; HLA-C*04:01; and HLA-C*02:02.

In preferred embodiments, the HLA-B and HLA-C alleles are selected from any one of the following HLA-B and HLA-C alleles: HLA-B*07:02 and HLA-C*07:02; HLA-B*08:01 and HLA-C*07:01; HLA-B*44:02 and HLA-C*05:01; HLA-B*35:01 and HLA-C*04:01; HLA-B*40:01 and HLA-C*03:04; HLA-B*57:01 and HLA-C*06:02; HLA-B*14:02 and HLA-C*08:02; HLA-B*15:01 and HLA-C*03:03; HLA-B*13:02 and HLA-C*06:02; HLA-B*44:03 and HLA-C*16:01; HLA-B*38:01 and HLA-C*12:03; HLA-B*18:01 and HLA-C*07:01; HLA-B*44:03 and HLA-C*04:01; HLA-B*51:01 and HLA-C*15:02; HLA-B*49:01 and HLA-C*07:01; HLA-B*15:01 and HLA-C*03:04; HLA-B*18:01 and HLA-C*12:03; HLA-B*27:05 and HLA-C*02:02; HLA-B*35:03 and HLA-C*04:01; HLA-B*18:01 and HLA-C*05:01; HLA-B*52:01 and HLA-C*12:02; HLA-B*51:01 and HLA-C*14:02; HLA-B*37:01 and HLA-C*06:02; HLA-B*53:01 and HLA-C*04:01; HLA-B*55:01 and HLA-C*03:03; HLA-B*44:02 and HLA-C*07:04; HLA-B*44:03 and HLA-C*07:01; HLA-B*35:02 and HLA-C*04:01; HLA-B*15:01 and HLA-C*04:01; and HLA-B*40:02 and HLA-C*02:02.

In embodiments, the T-cell further comprises a reduced or eliminated surface expression of HLA-B relative to an unmodified T cell by a genetic modification in the HLA-B gene and/or a reduced or eliminated surface expression of HLA-C relative to an unmodified T cell by a genetic modification in the HLA-C gene.

In further embodiments, the T-cell further comprises a reduced or eliminated surface expression of HLA-B relative to an unmodified T cell by a genetic modification in the HLA-B gene and the T cell is homozygous for HLA-C genotype.

In alternative embodiments, the T-cell further comprises a reduced or eliminated surface expression of HLA-C relative to an unmodified T cell by a genetic modification in the HLA-C gene and the T cell is homozygous for HLA-B genotype.

In further alternative embodiments, the T-cell further comprises a reduced or eliminated surface expression of HLA-B relative to an unmodified T cell by a genetic modification in the HLA-B gene and a reduced or eliminated surface expression of HLA-C relative to an unmodified T cell by a genetic modification in the HLA-C gene.

According to the invention, the engineered human T cell comprises a reduced or eliminated surface expression of HLA class II by a genetic modification in the CIITA gene.

In embodiments, the genetic modification in the CIITA gene comprises at least one nucleotide of at least one nucleotide of a splice site within the genomic coordinates chr16:10902171-chr16:10923242.

In embodiments, the genetic modification in the CIITA gene comprises a modification of at least one nucleotide of a splice acceptor site, preferably wherein the one nucleotide is A or G or T.

In embodiments, the genetic modification in the CIITA gene comprises a modification of a splice site boundary nucleotide.

In embodiments, the genetic modification in the CIITA gene comprises at least 5, 6, 7, 8, 9, or 10 contiguous nucleotides within the genomic coordinates chr16:10902171- chr16:10923242.

In embodiments, the genetic modification in the CIITA gene comprises at least one C to T substitution or at least one A to G substitution within the genomic coordinates chr16:10902171-chr16:10923242.

In embodiments, the genetic modification in the CIITA gene comprises an indel, a C to T substitution, or an A to G substitution within the genomic coordinates chosen from: chr16:10895410-10895430, chr16:10898649-10898669, chr16:10898658-10898678, chr16:10902171-10902191, chr16:10902173-10902193, chr16:10902174-10902194, chr16:10902179-10902199, chr16:10902183-10902203, chr16:10902184-10902204, chr16:10902644-10902664, chr16:10902779-10902799, chr16:10902788-10902808, chr16:10902789-10902809, chr16:10902790-10902810, chr16:10902795-10902815, chr16:10902799-10902819, chr16:10903708-10903728, chr16:10903713-10903733, chr16:10903718-10903738, chr16:10903721-10903741, chr16:10903723-10903743, chr16:10903724-10903744, chr16:10903873-10903893, chr16:10903878-10903898, chr16:10903905-10903925, chr16:10903906-10903926, chr16:10904736-10904756, chr16:10904790-10904810, chr16:10904811-10904831, chr16:10906481-10906501, chr16:10906485-10906505, chr16:10906486-10906506, chr16:10906487-10906507, chr16:10906492-10906512, chr16:10908127-10908147, chr16:10908130-10908150, chr16:10908131-10908151, chr16:10908132-10908152, chr16:10908137-10908157, chr16:10908138-10908158, chr16:10908139-10908159, chr16:10909006-10909026, chr16:10909007-10909027, chr16:10909018-10909038, chr16:10909021-10909041, chr16:10909022-10909042, chr16:10909172-10909192, chr16:10910165-10910185, chr16:10910176-10910196, chr16:10910186-10910206, chr16:10915547-10915567, chr16:10915551-10915571, chr16:10915552-10915572, chr16:10915567-10915587, chr16:10916348-10916368, chr16:10916359-10916379, chr16:10916362-10916382, chr16:10916449-10916469, chr16:10916450-10916470, chr16:10916455-10916475, chr16:10916456-10916476, chr16:10918423-10918443, chr16:10918504-10918524, chr16:10918511-10918531, chr16:10918512-10918532, chr16:10918539-10918559, chr16:10922153-10922173, chr16:10922478-10922498, chr16:10922487-10922507, chr16:10922499-10922519, chr16:10923205-10923225, chr16:10923214-10923234, chr16:10923218-10923238, chr16:10923219-10923239, chr16:10923220-10923240, chr16:10923221-10923241, and chr16:10923222-10923242.

In embodiments, the genetic modification in the CIITA gene comprises at least 5 contiguous nucleotides within the genomic coordinates chosen from: chr16:10906485-10906505, chr16:10906486-10906506, chr16:10906487-10906507, chr16:10906492-10906512, chr16:10908127-10908147, chr16:10908130-10908150, chr16:1090813l-10908151, chr16:10908132-10908152, chr16:10908137-10908157, chr16:10908138-10908158, chr16:10908139-10908159, chr16:10909006-10909026, chr16:10909007-10909027, chr16:10909018-10909038, chr16:10909021-10909041, chr16:10909022-10909042, chr16:10909172-10909192, chr16:10910165-10910185, chr16:10910176-10910196, chr16:10910186-10910206, chr16:10915547-10915567, chr16:10915551-10915571, chr16:10915552-10915572, chr16:10915567-10915587, chr16:10916348-10916368, chr16:10916359-10916379, chr16:10916362-10916382, chr16:10916449-10916469, chr16:10916450-10916470, chr16:10916455-10916475, chr16:10916456-10916476, chr16:10918423-109I8443, chr16:10918504-10918524, chr16:10918511-10918531, chr16:10918512-10918532, chr16:10918539-10918559, chr16:10922153-10922173, chr16:10922478-10922498, chr16:10922487-10922507, chr16:10922499-10922519, chr16:10923205-10923225, chr16:10923214-10923234, chr16:10923218-10923238, chr16:10923219-10923239, chr16:10923220-10923240, chr16:10923221-10923241, and chr16:10923222-10923242, preferably chr16:10908132-10908152, chr16:10908131-10908151, chr16:10916456-10916476, chr16:10918504-10918524, chr16:10909022-10909042, chr16:10918512-10918532, chr16:10918511-10918531, chr16:10895742-10895762, chr16:10916362-10916382, chr16:10916455-10916475, chr16:10909172-10909192, chr16:10906492-10906512, chr16:10909006-10909026, chr16:10922478-10922498, chr16:10895747-10895767, chr16:10916348-10916368, chr16:10910186-10910206, chr16:10906481-10906501, chr16:10909007-10909027, chr16:10895410-10895430, and chr16:10908130-10908150.

In embodiments, the genetic modification in the CIITA gene comprises at least 10 or at least 15 contiguous nucleotides within the genomic coordinates.

In embodiments, the genetic modification in the CIITA gene comprises an indel.

In embodiments, the genetic modification in the CIITA gene comprises at least one nucleotide of a splice site within the genomic coordinates chr16:10902171-chr16:10923242.

In embodiments, the genetic modification in the CIITA gene comprises a modification of at least one nucleotide of a splice acceptor site, preferably wherein the one nucleotide is A or G or T.

In embodiments, the genetic modification in the CIITA gene comprises a modification of a splice site boundary nucleotide.

In embodiments, the genetic modification in the CIITA gene comprises at least 5, 6, 7, 8, 9, or 10 contiguous nucleotides within the genomic coordinates chr16:10902171- chr16:10923242.

In embodiments, the genetic modification in the CIITA gene comprises at least one C to T substitution or at least one A to G substitution within the genomic coordinates chr16:10902171-chr16:10923242.

In embodiments, the genetic modification in the CIITA gene comprises an indel, a C to T substitution, or an A to G substitution within the genomic coordinates chosen from: chr16:10895410-10895430, chr16:10898649-10898669, chr16:10898658-10898678, chr16:10902171-10902191, chr16:10902173-10902193, chr16:10902174-10902194, chr16:10902179-10902199, chr16:10902183-10902203, chr16:10902184-10902204, chr16:10902644-10902664, chr16:10902779-10902799, chr16:10902788-10902808, chr16:10902789-10902809, chr16:10902790-10902810, chr16:10902795-10902815, chr16:10902799-10902819, chr16:10903708-10903728, chr16:10903713-10903733, chr16:10903718-10903738, chr16:10903721-10903741, chr16:10903723-10903743, chr16:10903724-10903744, chr16:10903873-10903893, chr16:10903878-10903898, chr16:10903905-10903925, chr16:10903906-10903926, chr16:10904736-10904756, chr16:10904790-10904810, chr16:10904811-10904831, chr16:10906481-10906501, chr16:10906485-10906505, chr16:10906486-10906506, chr16:10906487-10906507, chr16:10906492-10906512, chr16:10908127-10908147, chr16:10908130-10908150, chr16:10908131-10908151, chr16:10908132-10908152, chr16:10908137-10908157, chr16:10908138-10908158, chr16:10908139-10908159, chr16:10909006-10909026, chr16:10909007-10909027, chr16:10909018-10909038, chr16:10909021-10909041, chr16:10909022-10909042, chr16:10909172-10909192, chr16:10910165-10910185, chr16:10910176-10910196, chr16:10910186-10910206, chr16:10915547-10915567, chr16:10915551-10915571, chr16:10915552-10915572, chr16:10915567-10915587, chr16:10916348-10916368, chr16:10916359-10916379, chr16:10916362-10916382, chr16:10916449-10916469, chr16:10916450-10916470, chr16:10916455-10916475, chr16:10916456-10916476, chr16:10918423-10918443, chr16:10918504-10918524, chr16:10918511-10918531, chr16:10918512-10918532, chr16:10918539-10918559, chr16:10922153-10922173, chr16:10922478-10922498, chr16:10922487-10922507, chr16:10922499-10922519, chr16:10923205-10923225, chr16:10923214-10923234, chr16:10923218-10923238, chr16:10923219-10923239, chr16:10923220-10923240, chr16:10923221-10923241, and chr16:10923222-10923242.

In embodiments, the genetic modification in the CIITA gene comprises at least 5 contiguous nucleotides within the genomic coordinates chosen from: chr16:10906485-10906505, chr16:10906486-10906506, chr16:10906487-10906507, chr16:10906492-10906512, chr16:10908127-10908147, chr16:10908130-10908150, chr16:10908131-10908151, chr16:10908132-10908152, chr16:10908137-10908157, chr16:10908138-10908158, chr16:10908139-10908159, chr16:10909006-10909026, chr16:10909007-10909027, chr16:10909018-10909038, chr16:10909021-10909041, chr16:10909022-10909042, chr16:10909172-10909192, chr16:10910165-10910185, chr16:10910176-10910196, chr16:10910186-10910206, chr16:10915547-10915567, chr16:10915551-10915571, chr16:10915552-10915572, chr16:10915567-10915587, chr16:10916348-10916368, chr16:10916359-10916379, chr16:10916362-10916382, chr16:10916449-10916469, chr16:10916450-10916470, chr16:10916455-10916475, chr16:10916456-10916476, chr16:10918423-109I8443, chr16:10918504-10918524, chr16:10918511-10918531, chr16:10918512-10918532, chr16:10918539-10918559, chr16:10922153-10922173, chr16:10922478-10922498, chr16:10922487-10922507, chr16:10922499-10922519, chr16:10923205-10923225, chr16:10923214-10923234, chr16:10923218-10923238, chr16:10923219-10923239, chr16:10923220-10923240, chr16:10923221-10923241, and chr16:10923222-10923242, preferably chr16:10908132-10908152, chr16:10908131-10908151, chr16:10916456-10916476, chr16:10918504-10918524, chr16:10909022-10909042, chr16:10918512-10918532, chr16:10918511-10918531, chr16:10895742-10895762, chr16:10916362-10916382, chr16:10916455-10916475, chr16:10909172-10909192, chr16:10906492-10906512, chr16:10909006-10909026, chr16:10922478-10922498, chr16:10895747-10895767, chr16:10916348-10916368, chr16:10910186-10910206, chr16:10906481-10906501, chr16:10909007-10909027, chr16:10895410-10895430, and chr16:10908130-10908150.

In embodiments, the genetic modification in the CIITA gene comprises at least 10 or at least 15 contiguous nucleotides within the genomic coordinates.

According to the invention, the engineered human T cell comprises a switchable chimeric antigen cell surface receptor that comprises a tag-binding domain or a tag, an extracellular hinge and a transmembrane domain, and a signal transduction domain.

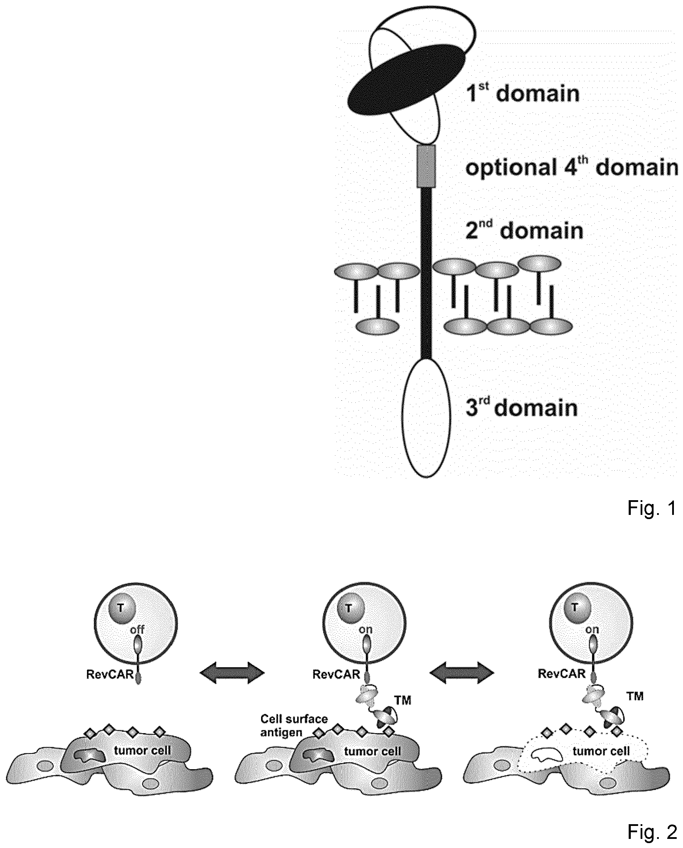

As used herein, the term "switchable chimeric antigen receptor" refers to an artificial chimeric fusion protein, in particular a receptor comprising a tag-binding domain or a tag, an extracellular hinge and a transmembrane domain and a signal transduction domain (Fig. 1). The domains can be derived from different sources and therefore, the receptor is called chimeric. Advantageously, the receptor can bind with the tag-binding domain or tag to different targeting modules.

In embodiments, the engineered human T cell comprises an exogenous nucleotide sequence encoding a switchable CAR that is expressed on the surface of the engineered T cell.

Advantageously, the engineered human T cell comprising a nucleotide sequence encoding a switchable CAR expresses the switchable CAR, has binding specificity for the tag-binding domain or tag of the targeting module, which in turn binds to an antigen on a target cell.

In preferred embodiments, the switchable chimeric antigen cell surface receptor is a reversed universal chimeric antigen cell surface receptor comprising a tag, an extracellular hinge and a transmembrane domain, and signal transduction domain.

Advantageously, engineered human T cells comprising a reversed universal chimeric antigen cell surface receptor are less exhausted and show a less differentiated phenotype after production.

As used herein, the term "tag" refers to a marker, in particular a peptide sequence or an organic molecule, attached to peptides or proteins to enable them to bind to specific atoms, ions or molecules, in particular the tag-binding domain.

In embodiments, the tag is selected from organic molecules including fluorescence labels, e.g. FITC (Fluorescein isothiocyanate), and biotin.

In embodiments, the tag is a peptide epitope tag. In further embodiments, the tag comprises 10 to 20 amino acids.

In embodiments, the peptide epitope tag is a myc-tag, a His-tag, a short linear peptide sequence from yeast transcription factor GCN4, preferably according to SEQ ID No. 7, SEQ ID No. 8 or mutants thereof; a leucine zipper sequence, preferably SYNZIP 1 to SYNZIP 48, BATF, FOS, ATF4, ATF3, BACH1, JUND, NFE2L3, HEPTAD (Reinke *et al.* 2010), a sequence according to SEQ ID No. 9 or SEQ ID No. 10 or mutants thereof; or a short linear peptide sequence from a human nuclear protein, preferably from the human La protein more preferably according to SEQ ID No. 11, SEQ ID No. 12, SEQ ID No. 13 or mutants thereof.

As used herein, the term "nuclear protein" refers to a protein found in the cell nucleus. Advantageously, tags, which are peptide sequences from nuclear antigens, cannot be accessed and bound by the corresponding tag-binding domain in the context of the native protein under physiological conditions. Further advantageously, the tag is not immunogenic. This leads to minimization of the risk of uncontrolled on-target off-site toxicities by CAR-expressing immune cells like the release of toxic levels of cytokines, referred to variously as cytokine storms or cytokine release syndrome (CRS).

In embodiments, the His-tag is an amino acid sequence consisting of histidine residues, preferably in the range of six to fourteen histidine residues.

In preferred embodiments, the peptide epitope tag is a myc-tag, a His-tag, a short linear peptide sequence from yeast transcription factor GCN4, preferably according to SEQ ID No. 7 or SEQ ID No. 8; a leucine zipper sequence, preferably SYNZIP 1 to SYNZIP 48, BATF, FOS, ATF4, ATF3, BACH1, JUND, NFE2L3, HEPTAD (Reinke *et al.* 2010), a sequence according to SEQ ID No. 9 or SEQ ID No. 10; or a short linear peptide sequence from a human nuclear protein, preferably from the human La protein more preferably according to SEQ ID No. 11, SEQ ID No. 12 or SEQ ID No. 13.

Preferably, the peptide epitope tag from the human La protein is the human La epitope E5B9 according to SEQ ID No. 11 or E7B6 according to SEQ ID No. 12 or SEQ ID No. 13, most preferably the human La epitope E5B9 according to SEQ ID No. 11 or E7B6 according to SEQ ID No. 13.

As used herein, the term "mutants" refers to peptides or proteins having at least 90 % sequence identity to the named peptides or proteins, preferably at least 95 % sequence identity. Advantageously, the mutants are capable of having one or more activities of the named peptides or proteins, in particular bind the identical tag-binding domains as the peptide epitope tag.

In embodiments, mutants are truncated versions of peptides or proteins. As used herein, the term "truncated versions" refers to shortened peptides or proteins having at least 90 % sequence identity to the named peptides or proteins, preferably at least 95 % sequence identity, more preferably having a chain length of at least 90 % and a sequence identity of 100 %, most preferably a chain length of at least 95 % and a sequence identity of 100 %. Advantageously, the truncated version has at least 90 %, preferably of at least 95 %; of the activity of the named peptide or protein.

In embodiments, the tag-binding domain is an antibody, antibody fragment, a protein or a peptide.

As used herein, the term "antibody" refers to a protein, which binds antigens via the antigen-binding fragment variable region (Fab). This is composed of one constant and one variable domain of each of the heavy (V_{H}) and the light chain (V_{L}). As used herein, the term "antibody fragment or antigen-binding fragment" refers to a protein comprising at least the V_{L} or V_{H} of an antibody. In an embodiment, antibody fragments are selected from single-chain variable fragments (scFv), single-chain antibodies, F(ab')2 fragments, Fab fragments, and fragments produced by a Fab expression library or single-domain antibodies (nanobodies).

As used herein, the term "single-chain variable fragment (scFv)" refers to an artificial antibody fragment comprising a variable domain of a light chain and a variable domain of a heavy chain of an antibody covalently linked. In embodiments, the V_{L} and V_{H} of an antibody are covalently linked by a short peptide of 10 to 25 amino acids. In further embodiments, the short peptide links the N-terminus of the V_{H} with the C-terminus of the V_{L}, or vice versa.

Preferably, V_{H} and V_{L} are connected via a glycine-serine linker with the structure (GₓS_{y}) with x and y selected from 1 to 10, preferably 1 to 5. Mostly preferred are 1 to 10 repeats of the sequence G₄S₁ (SEQ ID No. 4). Moreover, linkers are preferred that are constituted of a peptide sequence that can increase the protease resistance of the antibody derivatives. As used herein, the term "derivative" refers to a molecule having a high degree of structural identity to the molecule, preferably the same scaffold, wherein at least one atom, group of atoms, functional group or substructure is replaced with another atom, group of atoms functional group or substructure, e.g. a hydroxy group. Advantageously, the derivative is capable of having one or more activities of the named molecule.

In embodiments, the linker is SEQ ID No. 5 or SEQ ID No. 6.

In embodiments, the tag-binding domain is an antibody or an antibody fragment, a protein or a peptide binding to a myc-tag, a His-tag, a short linear peptide sequence from yeast transcription factor GCN4, a leucine zipper sequence or a short linear peptide sequence from a human nuclear protein, preferably from the human La protein.

In preferred embodiments, the tag-binding domain is an antibody or an antibody fragment.

In embodiments, the antibody is obtained from an animal species, preferably from a mammal such as human, simian, mouse, rat, rabbit, guinea pig, horse, cow, sheep, goat, pig, dog or cat. Preferably, the antibody or antibody fragment is a human, humanized or deimmunized antibody. Humanized antibodies can be prepared in various ways, for example, by resurfacing and CDR grafting. In case of resurfacing, a combination of molecular modeling, statistical analyses, and mutagenesis is used to modify all non-CDR regions on the surface of the antibody to become similar to the surface of antibodies of the target organism. In CDR grafting, the CDR regions according to the invention are introduced into known human framework regions, which are similar in sequence to the original ones. Deimmunized antibodies can be obtained by specifically mutating residues that confer immunogenicity hotspots as predicted based on *in silico* peptide-MHC affinity prediction.

As used herein, the term "CDR (Complementarity-determining regions)" refers to parts of the variable chains in antibodies or antibody fragments, where the antibodies or antibody fragments bind to their specific antigen. An antibody comprises three CDRs (CDR1, CDR2 and CDR3), arranged non-consecutively, on the amino acid sequence of each variable domain and thus, six CDRs on the two variable domains (V_{H} and V_{L}), which can come into contact with the antigen.

In embodiments, the antibody or antibody fragment is a polyclonal, a monoclonal or a chimeric antibody, wherein an antigen-binding region of a non-human antibody is transferred into the framework of a human antibody by recombinant DNA techniques including *in silico* design.

In embodiments, antibodies to a selected tag or antigen may be produced by immunization of various hosts including, but not limited to, goats, rabbits, rats, mice, humans, through injection with cells expressing a particular protein, DNA or RNA encoding for the protein, the protein itself or any portion, fragment or oligopeptide that retain immunogenic properties of the protein.

In embodiments, the tag-binding domain binds to a tag from the human nuclear La protein; preferably, the tag-binding domain is an antibody or an antigen-binding fragment, comprising a V_{L} according to the following sequence:
DIVMTQSPDSLAVSLGERATINCX₂₄SSQSLLNSRTX₃₅KNYLAWYQQKPGQPPKLLIYWASTR X₆₁SGVPDRFSGSGSGTDFTLTISSLQAEDVAVYYCKQSYNLX₁₀₁TFGGGTKVEIK (SEQ ID No. 14), wherein X₂₄, X₃₅, X₆₁ and X₁₀₁ are independently from each other selected from a proteinogenic alpha-amino acid residue;
or a sequence having at least 90 % sequence identity, preferably at least 95 % sequence identity; to one of the sequences SEQ ID No. 16 or SEQ ID No. 18.

In embodiments, the tag-binding domain constitutes an anti-La epitope scFv.

In some embodiments, X₂₄ to X₁₀₁ are selected as follows:
X₂₄ is selected from polar and/or positive charged residues, such as Serine, Threonine, Asparagine, Glutamine, Histidine, Lysine and Arginine; preferably Lysine or Arginine;
X₃₅ is preferably selected from Lysine and Proline;
X₆₁ is selected from polar and charged residues, such as Asparagine, Aspartic Acid, Glutamine, Glutamic acid, Histidine, Lysine and Arginine, preferably Glutamic acid and Lysine;
X₁₀₁ is selected from hydrophobic residues, such as Isoleucine, Leucine, Valine, Alanine, Methionine, Phenylalanine, Proline and Tryptophan; preferably Leucine or Proline.

In further embodiments, the tag-binding domain is an antibody or an antigen-binding fragment comprising a sequence having at least90 % sequence identity, preferably at least 95 % sequence identity; to one of the sequences SEQ ID No. 15 or SEQ ID No. 17.

Preferably, the tag-binding domain comprises a sequence having each at least 90 % sequence identity, preferably at least 95 % sequence identity; to the sequences according to SEQ ID NO. 15 (V_{H}) and SEQ ID NO. 16 (V_{L}) or the sequences according to SEQ ID NO. 17 (V_{H}) and SEQ ID NO. 18 (V_{L}).

Most preferably, the tag-binding domain constitutes the anti-La 5B9 scFv according to SEQ ID NO. 15 (V_{H}) and SEQ ID NO. 16 (V_{L}) or the anti-La 7B6 scFv according to SEQ ID NO. 17 (V_{H}) and SEQ ID NO. 18 (V_{L}).

As used herein, the term "extracellular hinge and transmembrane domain" refers to a flexible peptide sequence connected to the tag-binding domain or tag, which anchors the switchable CAR into the cell membrane of the cell and protrudes from the surface of the cell for optimal binding to its particular targeting module.

In embodiments, the extracellular hinge and transmembrane domain are selected from hinge and transmembrane domains of human CD28 molecule, CD8a chain, NK cell receptors, preferably natural killer group NKG2D; or parts of the constant region of an antibody and combinations thereof. As used herein, the term "combinations thereof' refers to combinations of the different hinge and transmembrane domains.

Examples of combinations of the extracellular hinge and transmembrane domain are, but are not limited to, CD28 extracellular hinge and transmembrane domain, CD8alpha extracellular hinge and transmembrane domain, IgG1 or IgG4 constant regions combined with a CD8alpha, CD28 or CD137 transmembrane domain.

In preferred embodiments, the extracellular hinge and transmembrane domain comprises an extracellular domain of CD28, more preferably the extracellular domain, in which the B7 binding site was mutated. Advantageously, the mutation of the B7 binding sites results in an abrogated ligand binding. In further preferred embodiments, the extracellular hinge and transmembrane domain comprises a CD28 transmembrane domain.

As used herein, the term "signal transduction domain" refers to a peptide sequence which transmits a signal into the cell by cross-linkage of the cell expressing the switchable CAR (effector cell) to a human cell surface protein or protein complex (target cell). Cross-linkage between effector and target cell is mediated by the targeting module according to the invention.

In embodiments, the signal transduction domain is selected from cytoplasmic regions of CD28, CD137 (4-1BB), CD134 (OX40), CD278 (ICOS), DAP10 and CD27, programmed cell death-1 (PD-1), cytotoxic T-lymphocyte antigen 4 (CTLA-4), cytoplasmic regions of CD3 chains, DAP12, CD122 (interleukin-2 receptor β), CD132 (interleukin-2 receptor γ), CD127 (interleukin-7 receptor α), CD360 (interleukin-21 receptor), activating Fc receptors, mutants thereof and combinations thereof.

As used herein, the term "mutants" refers to proteins having at least 90 % sequence identity to the signal transduction domains, preferably at least 95 % sequence identity. Advantageously, the mutant transmits a signal into the cell by cross-linkage of the cell expressing the switchable CAR (effector cell) to a human cell surface protein or protein complex (target cell) in the same way as the named signal transduction domains.

In embodiments, mutants are truncated versions. As used herein, the term "truncated versions" refers to shortened proteins having at least 90 % sequence identity to the signal transduction domains, preferably at least 95 % sequence identity, more preferably having a chain length of at least 90 % and a sequence identity of 100 %, most preferably a chain length of at least 95 % and a sequence identity of 100 %. Advantageously, the truncated version has an activity of at least 90 %, more preferably of at least 95 %; of the named signal transduction domains.

Guedan *et al.* describes the use of a mutant of cytoplasmic regions of CD28 as signal transduction domain (Guedan *et al.* 2020).

In preferred embodiments, the signal transduction domain is selected from cytoplasmic regions of CD28, CD137 (4-1BB), CD134 (OX40), CD278 (ICOS), DAP10 and CD27, programmed cell death-1 (PD-1), cytotoxic T-lymphocyte antigen 4 (CTLA-4), cytoplasmic regions of CD3 chains, DAP12, CD122 (interleukin-2 receptor β), CD132 (interleukin-2 receptor γ), CD127 (interleukin-7 receptor a) and CD360 (interleukin-21 receptor) activating Fc receptors.

In mostly preferred embodiments, the signal transduction domain comprises a CD28 intracellular domain, more preferably a CD28 intracellular domain, in which the internalization motif was mutated, fused to the CD3ζ intracellular domain.

In embodiments, the switchable chimeric antigen receptor comprises at least one signal transduction domain, preferably two, three, four or more signal transduction domains, especially preferably selected from cytoplasmic regions of CD28, CD137, CD134, CD278, DAP10 and CD27, PD-1, CTLA-4, cytoplasmic regions of CD3 chains, DAP12, CD122, CD132, CD127, CD360, activating Fc receptors, mutants thereof and combinations thereof.

In preferred embodiments, the switchable chimeric antigen receptor comprises at least one signal transduction domain, preferably two, three, four or more signal transduction domains, especially preferably selected from cytoplasmic regions of CD28, CD137, CD134, CD278, DAP10 and CD27, PD-1, CTLA-4, cytoplasmic regions of CD3 chains, DAP12, CD122, CD132, CD127, CD360 and activating Fc receptors.

In further embodiments, the switchable chimeric antigen receptor comprises a further domain, wherein the further domain is a short peptide linker in the extracellular portion of the receptor that may serve to detect the chimeric antigen receptor on the cell surface or stimulate the chimeric antigen receptor T cell.

In preferred embodiments, the further domain forms a linear epitope for a monoclonal antibody (mab) specifically binding to the further domain. In some embodiments, the further domain comprises at least one linear epitope, preferably E7B6 according to SEQ ID No. 12 or SEQ ID No. 13.

In some embodiments, the further domain is located in between the tag-binding domain or the tag and the extracellular hinge domain or an integral part of the extracellular hinge domain.

Advantageously, the switchable CAR engrafted cells with the further domain can be specifically stimulated to proliferate preferentially and persist longer compared to non-engrafted cells either *in vitro* or *in vivo.* Further advantageously, the further domain may be also used to purify switchable CAR engrafted cells from mixed cell populations or to dampen switchable CAR engrafted cell-mediated immune response and to eliminate switchable CAR engrafted cells *in vivo.*

In further embodiments, the switchable CAR comprises a signal peptide. Advantageously, the signal peptide allows for expression on the cell surface of an effector cell. In embodiments, the signal peptide is located at the N-terminus of the switchable CAR nucleotide sequence in front of the tag-binding domain or the tag. In some embodiments, the signal peptide targets proteins to the secretory pathway either co-translationally or post-translationally and is selected from leader peptides from proteins like CD28, CD8alpha, IL-2, lysozyme C or the heavy or light chains of antibodies of human origin to avoid immunogenic reactions.

In embodiments, the tag-binding domain or tag is present at the amino-terminal end of the polypeptide that comprises the switchable CAR. Advantageously, locating the tag-binding domain or tag at the amino terminus permits the tag-binding domain or tag unhampered access to the targeting module that is bound to the target cell.

In embodiments, the switchable CAR comprises a sequence according to one of the sequences SEQ ID No. 33 to SEQ ID No. 36. Preferably, the switchable CAR has a sequence according to one of the sequences SEQ ID No. 33 to SEQ ID No. 36.

In preferred embodiments, the switchable CAR comprises a sequence according to SEQ ID No. 33. More preferably, the switchable CAR has a sequence according to SEQ ID No. 33.

In embodiments, the engineered human T cell is used in a method for stimulating a chimeric antigen receptor-mediated immune response in a mammal, preferably in the treatment of cancer, infectious disease or autoimmune disease.

The term "autoimmune disorder" refers to an abnormal immune response of the body against substances and tissues normally present in the body (autoimmunity).

In embodiments, the engineered human T cell is used in the combination with a targeting module comprising a tag-binding domain or a tag and at least one target cell-binding domain or a nucleic acid, vector or cell encoding the targeting module, wherein the tag-binding domain of the targeting module binds to the tag of the switchable chimeric antigen receptor or the tag of the targeing module binds to the tag-binding domain of the switchable chimeric antigen receptor, wherein the engineered human T cell and the targeting module are administered in combination.

As used herein, the term "administered in combination" refers to a treatment, wherein the targeting module is administered prior to, simultaneously with and/or after the administration of the vector or cell comprising a nucleotide sequence encoding a switchable chimeric antigen receptor.

In embodiments, the targeting module is administered one hour to 2 days, preferably 4 to 24 hours, prior to the administration of the engineered human T cell. Advantageously, the administration of the targeting module prior to the administration of the engineered human T cell stimulates the switchable chimeric antigen receptor and increases the expansion of the T cells and their accumulation at the target site.

In further embodiments, the engineered human T cell is administered simultaneously with the targeting module.

In further embodiments, the targeting module is administered until, preferably in the range of 3 days to 30 days, after the administration of the engineered human T cell. Furthermore, additional such doses of the targeting module may be administered following resting periods to reactivate the switchable CAR-carrying effector cells.

A further aspect of the invention provides a pharmaceutical composition comprising the engineered human T cell according to the invention and a pharmaceutical acceptable thinner or carrier.

The pharmaceutical composition is preferably administered parenterally, particularly preferred intravenously. In embodiments, the pharmaceutical composition is present in a form suitable for intravenous administration. Preferably, the pharmaceutical composition is a solution, emulsion or suspension.

In embodiments, the pharmaceutical composition is an injectable buffered solution comprising a concentration in the range of 1•10⁵ to 1•10⁸ per mL of the engineered human T cell.

The pharmaceutical composition comprises a pharmaceutically acceptable thinner (dilution agent) or carrier. In embodiments, the carrier is selected from water, an aqueous buffer solution, 0.9 % saline solution, 5 % glucose, 5 % xylitol, 0.3 % glycine solution, ringer solutions or amino acid solutions. In further embodiments, the aqueous buffer solution is selected from an aqueous histidine, sodium succinate, sodium citrate, sodium phosphate or potassium phosphate-buffered solution with a pH value in the range of pH 5.0 to pH 7.0. In embodiments, the aqueous buffer solution has a buffer concentration in the range of 1 mmol/I (mM) to 500 mM, preferably in the range of 5 mM to 20 mM, especially preferred in the range of 5 mM to 10 mM.

In embodiments, the carrier comprises sodium chloride, preferably in a concentration in the range of 1 mM to 300 mM, especially preferred 150 mM.

In embodiments, the pharmaceutical composition further comprises a stabilizer, preferably with a concentration in the range of 1 mM to 900 mM, especially preferred in the range of 50 mM and 600 mM. In embodiments, the stabilizer is sucrose, trehalose or L-methionine.

In some embodiments, the pharmaceutical composition further comprises pharmaceutically acceptable excipients. The term "pharmaceutically acceptable excipients" refers to compounds, which provide approximately physiological conditions and/or increase the stability, such as agents for adjusting the pH value and buffering agents, agents for adjusting the toxicity and the like. In embodiments, pharmaceutically acceptable excipients are selected from sodium acetate, sodium chloride, potassium chloride, calcium chloride, sodium lactate and polysorbate-80, preferably polysorbate-80 in the range of 0.0001 % (w/v) to 1 % (w/v), especially preferred in the range of 0.001 % (w/v) to 0.1 % (w/v).

In further embodiments, the pharmaceutical composition is sterile. The pharmaceutical composition is sterilized by conventional well-known techniques including, but not limited to, sterile filtration.

In embodiments, the pharmaceutical composition is used for administration to a subject.

In embodiments, the pharmaceutical composition is lyophilized prior to storage or stored as solution at ambient temperature or below, including, but not limited to, frozen storage.

In embodiments, the pharmaceutical composition is reconstituted and/or diluted in an infusion and stabilizer solution prior to administration to a subject. The solutions used for reconstitution or infusion/stabilization may contain any of the components mentioned for the pharmaceutical composition or similar components.

In embodiments, the pharmaceutical composition is used in a method for stimulating a chimeric antigen receptor-mediated immune response in a mammal, preferably in the treatment of cancer, infectious disease or autoimmune disease.

In embodiments, the pharmaceutical composition is used in the treatment of cancer, infectious disease or autoimmune disease, wherein the engineered human T cell is matched for the HLA-B and HLA-C alleles to a patient.

A further aspect of the invention is a population of cells comprising the engineered human T cell according to the invention.

In embodiments, the population of cells is at least 65%, preferably at least 80%, more preferably at least 90%, even more preferably at least 95%, or most preferably at least 99% TRAC, HLA-A and/or TCR protein negative as measured by flow cytometry.

Another aspect of the invention is a kit comprising
a) an engineered human T cell, comprising:
   i. reduced or eliminated surface expression of endogenous T cell receptor alpha chain by a genetic modification in the T cell receptor alpha chain gene,
   ii. reduced or eliminated surface expression of HLA-A relative to an unmodified T cell by a genetic modification in the HLA-A gene,
   iii. reduced or eliminated surface expression of HLA class II by a genetic modification in the CIITA gene, and
   iv. a switchable chimeric antigen cell surface receptorthat comprises:
      - a tag-binding domain or a tag,
      - an extracellular hinge and a transmembrane domain, and
      - a signal transduction domain, and
b) a targeting module comprising a tag-binding domain or a tag and at least one target cell-binding domain or a nucleic acid, vector or cell encoding the targeting module,
   wherein the tag-binding domain of the targeting module binds to the tag of the switchable chimeric antigen receptor or the tag of the targeting module binds to the tag-binding domain of the switchable chimeric antigen receptor.

As used herein, the term "targeting module" refers to a polypeptide or protein with at least two different domains, wherein each domain is specific for a target or a uniform group of targets, respectively, wherein at least one domain is specific for a target cell and one domain is specific for a switchable chimeric antigen receptor, in particular the tag-binding domain or the tag. In embodiments, the targeting module is isolated. As used herein, the term "isolated" means altered or removed from the natural state.

Preferably, the targeting module is expressed as a recombinant protein. In further embodiments, the targeting module is chemically synthesized.

As used herein, the term "specific" refers to the ability of an antibody or antibody fragment or a protein, peptide or low molecular weight organic ligand to recognize and bind with a binding partner (e.g. a tumor antigen) protein present in a sample, but not substantially recognize or bind other molecules in the sample.

As used herein, the term "binds" or "binding" refers to a non-covalent binding, in particular ionic bonds, hydrogen bonds, Van der Waals forces and/or hydrophobic interactions.

In embodiments, the targeting module is in monomeric, dimeric or polymeric form, preferably in monomeric form.

In further embodiments, the targeting module is monovalent, bivalent or multivalent.

As used herein, the term "target cell-binding domain" refers to a peptide, protein, or low molecular weight organic ligand, which specifically binds a protein or protein complex (antigen) on the surface of a target cell, preferably a cancer cell, T cell, infected cell, pathogens or parasites.

As used herein, the terms "peptide", "polypeptide" and "protein" are used interchangeably, and refer to a compound comprised of amino acid residues covalently linked by peptide bonds. A protein or peptide must contain at least two amino acids, and no limitation is placed on the maximum number of amino acids that can comprise a protein's or peptide's sequence.

As used herein, the term "low molecular weight organic ligand" refers to an organic molecule with a molecular weight of maximal 10 kilodaltons, preferably of maximal 3 kilodaltons, which specifically binds a protein or protein complex (antigen) on the surface of a target cell, preferably a cancer cell, T cell, infected cell or pathogens or parasites.

In embodiments, the at least one target cell-binding domain is an antibody, antibody fragment, a protein, a peptide or a low molecular weight organic ligand that binds to surface antigens selected from the group comprising CD2, CD3, CD4, CD5, CD7, CD8, CD10, CD15, CD19, CD20, CD22, CD23, CD25, CD30, CD33, CD38, CD44, CD44v6 CD52, CD66a, CD66b, CD66c, CD66d, CD66e, CD66f, CD90, CD99, CD123, CD133, CD135, CD150 CD181, CD182, CD184, CD223, CD229, CD269, CD273, CD274, CD276, CD279, CD319, CD366 and CD371,cytokine receptors, preferably interleukin receptors, especially preferred IL-8Rα, IL-8Rβ, IL-11Rα, IL-11Rβ, IL13Rα1; CXCR4, c-Met, mesothelin, members of the epidermal growth factor receptor family and mutants thereof, especially preferred ErbB1, ErbB2, ErbB3, ErbB4 or mutants thereof; members of the tumor necrosis factor receptor superfamily, claudins, ephrins, ephrin receptors, especially preferred EphA1-10, EphA5 or EphB1-6; fucosyl transferases, prostate specific antigens, preferably PSCA and PSMA; embryonic antigens, preferably CEA and fetal acethylcholine receptor; members of the vascular endothelia growth factor family, EpCAM, AFP, members of the intercellular adhesion molecule family, C-type lectins, integrins, members of the mucin protein family, FSHR, HMW-MAA, FBP, folate receptors, somatostatin receptors, ligands of the NKG2D receptor, members of the epithelia glycoprotein family, diasialogangliosides, glypicans, G protein-coupled receptors, human papillomavirus proteins, cancer-testis antigens, fibroblast activation proteins, members of the carbonic anhydrase family, members of the carbohydrate antigen family, Notch ligands, MCSP, glycoprotein A33, guanylate cyclase 2C and tumor-specific glycans, including mutants and analogues of the named antibodies, antibody fragments, proteins, peptides or low molecular weight organic ligands.

As used herein, the term "mutants" refers to peptides or proteins having at least 90 % sequence identity to the named antibodies, antibody fragments, proteins or peptides, preferably at least 95 % sequence identity. Advantageously, the mutants bind the identical antigens as the named antibodies, antibody fragments, proteins or peptides.

As used herein, the term "analogues" refers to molecules having a high degree of structural identity to the named antibodies, antibody fragments, proteins, peptides or low molecular weight organic ligands, preferably at least one atom, group of atoms, functional group or substructure is replaced with another group of atoms, e.g. a hydroxy group. In embodiments, an analogue of somatostatin (SRIF14) is octreotide or pasireotide. Advantageously, the analogues bind the identical antigens as the named antibodies, antibody fragments, proteins, peptides or low molecular weight organic ligands.

In embodiments, the analogues of the named antibodies, antibody fragments, proteins or peptides comprise modifications selected from the group comprising D amino acids, pseudo peptide bonds, aminoalcohols, non-proteinogenic amino acids, unnatural amino acids, amino acids with modified side chains and/or circular proteins. Advantageously, these analogues reveal increased stability.

The term "target cell-binding domain" also comprises soluble T cell receptors, which are composed of the alpha and beta or the gamma and delta chains of a T cell receptor (TCR), fragments or mutants thereof. Such TCR-derived binding moieties recognize and bind to peptides presented by human leukocyte antigen class (HLA) I and II protein complexes. Examples are, but are not limited to, TCRs specific for peptides derived from proteins like EGFR family, survivin, sry-like high motility group box (SOX) protein family, melanoma-associated antigens (e.g. autoimmunogenic cancer/testis antigen NY-ESO-1, members of the melanoma antigen family A MAGEA, the preferentially expressed antigen in melanoma PRAME), and leukemia-associated antigens (e.g. Wilms tumor gene 1 WT1).

In further embodiments, the target cell-binding domain is a soluble T cell receptor consisting of the alpha and beta or the gamma and delta chain of a T cell receptor (TCR).

In embodiments, the at least one target cell-binding domain is an antibody, antibody fragment, a protein, a peptide or a low molecular weight organic ligand that binds to surface antigens selected from the group comprising CD2, CD3, CD4, CD5, CD7, CD8, CD10, CD15, CD19, CD20, CD22, CD23, CD25, CD30, CD33, CD38, CD44, CD44v6 CD52, CD66a, CD66b, CD66c, CD66d, CD66e, CD66f, CD90, CD99, CD123, CD133, CD135, CD150 CD181, CD182, CD184, CD223, CD229, CD269, CD273, CD274, CD276, CD279, CD319, CD366 and CD371, cytokine receptors, CXCR4, c-Met, mesothelin, members of the epidermal growth factor receptor family and mutants thereof, members of the tumor necrosis factor receptor superfamily, claudins, ephrins, ephrin receptors, fucosyl transferases, prostate specific antigens, embryonic antigens, members of the vascular endothelia growth factor family, EpCAM, AFP, members of the intercellular adhesion molecule family, C-type lectins, integrins, members of the mucin protein family, FSHR, HMW-MAA, FBP, folate receptors, somatostatin receptors, ligands of the NKG2D receptor, members of the epithelia glycoprotein family, diasialogangliosides, glypicans, G protein-coupled receptors, human papillomavirus proteins, cancer-testis antigens, fibroblast activation proteins, members of the carbonic anhydrase family, members of the carbohydrate antigen family, Notch ligands, MCSP, glycoprotein A33, guanylate cyclase 2C and tumor-specific glycans.

In preferred embodiments, the at least one target cell-binding domain is an antibody, antibody fragment, a protein, a peptide or a low molecular weight organic ligand that binds to CD123.

In embodiments, the variable regions of the at least one target cell-binding domain, preferably the CD123-binding domain; comprises a humanized amino acid sequence.

In more preferred embodiments, the at least one target cell-binding domain is an antibody fragment that binds to CD123.

In some embodiments, the targeting module according to the invention is bivalent or multivalent and comprises at least one CD123-binding domain.

In embodiments, the different domains of the targeting module are linked with each other by a linker. The linker comprises a short sequence of preferably 20 to 30 amino acid residues. In embodiments, the targeting module comprises a flexible peptide sequence that is selected such that the domains have a three-dimensional folding that allows them to exhibit the specificity for effector cell and target cell binding.

Preferred linkers are glycine-serine linkers with the structure (GₓS_{y}) with x and y selected from 1 to 10, preferably 1 to 5. Mostly preferred are 2 to 5 repeats of the sequence G₄S₁ (SEQ ID No. 4). Moreover, linkers are preferred that are constituted of a peptide sequence that can increase the protease resistance of the antibody derivatives.

In embodiments, the linker of the tag-binding domain comprises 20 to 30 amino acids, preferably 25 amino acids.

In embodiments, the linker has a sequence according to SEQ ID No. 5 or SEQ ID No. 6.

In embodiments, the targeting module comprises one of the sequences according to SEQ ID No. 19 to SEQ ID No. 32.

In preferred embodiments, the targeting module comprises one of the sequences according to SEQ ID No. 21, SEQ ID No. 22, SEQ ID No. 24, SEQ ID No. 25, SEQ ID No. 28, SEQ ID No. 30, SEQ ID No. 31 or SEQ ID No. 32. More preferably, the targeting module comprises one of the sequences according to SEQ ID No. 24 or SEQ ID No. 31. Mostly preferred, the targeting module has one of the sequences according to SEQ ID No. 24 or SEQ ID No. 31.

In embodiments, the targeting module comprises a further domain selected from the group comprising co-stimulatory ligands, radionuclides, cell death-inducing chemical compounds and half-life increasing domains, preferably IgG1 Fc, IgG2 Fc, IgG3 Fc, IgG4 Fc, HSA, FcRn-binding peptides or mutants thereof. As used herein, the term "mutants" refers to proteins having at least 90% sequence identity to the half-life increasing domain, preferably at least 95% sequence identity. Advantageously, the mutant is capable of having one or more activities of the named peptides or proteins; in particular, the mutant increases the half-life like the half-life increasing domain.

In preferred embodiments, the targeting module comprises a further domain selected from the group comprising co-stimulatory ligands, radionuclides, cell death-inducing chemical compounds and half-life increasing domains, preferably IgG1 Fc, IgG2 Fc, IgG3 Fc, IgG4 Fc, HSA or FcRn-binding peptides.

In embodiments, the length of the targeting module is in the range of 20 to 1600 amino acids, preferably 200 to 800 amino acids.

In embodiments, the kit further comprises at least one further targeting module or at least one further nucleic acid, vector or cell encoding a further targeting module,
wherein the at least one further targeting module comprises at least one target cell-binding domain and a tag-binding domain or a tag,
wherein the at least one target cell-binding domain is an antibody, antibody fragment, a protein, a peptide or a low molecular weight organic ligand that binds to surface antigens selected from the group comprising CD2, CD3, CD4, CD5, CD7, CD8, CD10, CD15, CD19, CD20, CD22, CD23, CD25, CD30, CD33, CD38, CD44, CD44v6 CD52, CD66a, CD66b, CD66c, CD66d, CD66e, CD66f, CD90, CD99, CD133, CD135, CD150 CD181, CD182, CD184, CD223, CD229, CD269, CD273, CD274, CD276, CD279, CD319, CD366 and CD371, cytokine receptors, preferably interleukin receptors, especially preferred IL-8Rα, IL-8Rβ, IL-11Rα, IL-11Rβ, IL13Rα1; CXCR4, c-Met, mesothelin, members of the epidermal growth factor receptor family and mutants thereof, especially preferred ErbB1, ErbB2, ErbB3, ErbB4 or mutants thereof; members of the tumor necrosis factor receptor superfamily, claudins, ephrins, ephrin receptors, especially preferred EphA1-10, EphA5 or EphB1-6; fucosyl transferases, prostate specific antigens, preferably PSCA and PSMA; embryonic antigens, preferably CEA and fetal acethylcholine receptor; members of the vascular endothelia growth factor family, EpCAM, AFP, members of the intercellular adhesion molecule family, C-type lectins, integrins, members of the mucin protein family, FSHR, HMW-MAA, FBP, folate receptors, somatostatin receptors, ligands of the NKG2D receptor, members of the epithelia glycoprotein family, diasialogangliosides, glypicans, G protein-coupled receptors, human papillomavirus proteins, cancer-testis antigens, fibroblast activation proteins, members of the carbonic anhydrase family, members of the carbohydrate antigen family, Notch ligands, MCSP, glycoprotein A33, guanylate cyclase 2C and tumor-specific glycans, including mutants and analogues of the named antibodies, antibody fragments, proteins, peptides or low molecular weight organic ligands,
wherein the targeting module and the at least one further targeting module comprise different target cell-binding domains, and identical tag-binding domains or a tags.

In further embodiments, the at least one further targeting module comprises two target cell-binding domains binding to surface antigens selected from PSCA and PSMA, ErbB-1 and ErbB-2, PSCA and ErbB-2, PSMA and CEA, IL13Ra2 and ErbB-2, CD38 and CD269, CD19 and CD20, mesothelin and mucin 16, PD-L1 and ErbB-2.

In embodiments, the kit comprises one to three targeting modules, preferably one targeting module binding to CD123 and one or two further targeting modules.

In alternative embodiments, the kit comprises a nucleic acid, vector or cell encoding the targeting module. The nucleic acid, vector and/or cell are isolated.

In embodiments, the nucleic acid is a cDNA. As used herein, the term "cDNA" (complementary DNA) refers to double-stranded DNA synthesized from a single-stranded RNA, e.g. mRNA, in a reaction catalyzed by the enzyme reverse transcriptase. In embodiments, cDNA is of synthetic origin. In further embodiments, cDNA is derived from mRNA, therefore containing only exons but no introns, as opposed to genomic DNA.

The vector is preferably a plasmid, an artificial chromosome, linearized DNA or RNA, a virus particle or another vector that contains an expression cassette that is incorporated stably into the genome of a host cell or host organism.

In embodiments, the cell is selected from immune cells, preferably with cytolytic, phagocytic or immunosuppressive activity, such as T cells, Natural Killer (NK) cells and macrophages. In preferred embodiments, the cell is selected from T cells, including alpha/beta and gamma/delta T cells or subpopulations of T cells like stem-cell memory T cells or central memory T cells, cytotoxic T cells or NK cells.

In embodiments, the engineered human T cell and/or the targeting module are in the form of a pharmaceutical composition.

In embodiments, the kit is used in a method for stimulating a chimeric antigen receptor-mediated immune response in a mammal, preferably in the treatment of cancer, infectious disease or autoimmune disease.

In embodiments, the engineered human T cell and/or the targeting module are used for preparing a medication for therapeutic and/or diagnostic use in case of cancer, an infection or an autoimmune disease.

In embodiments, the engineered human T cell and/or the targeting module are used for treating CD123-expressing tumors, preferably acute myeloid leukemia.

A further aspect of the invention is a kit comprising
a) a CRISPR system comprising
   - a nucleic acid that causes downregulation of gene expression of an endogenous T cell receptor alpha chain,
   - a nucleic acid that causes downregulation of gene expression of HLA-A,
   - a nucleic acid that causes downregulation of gene expression of CIITA, and
b) a nucleic acid or a vector comprising a nucleic acid encoding a switchable chimeric antigen cell surface receptor that comprises:
   - a tag-binding domain or a tag,
   - an extracellular hinge and a transmembrane domain, and
   - a signal transduction domain, and
c) a targeting module comprising a tag-binding domain or a tag and at least one target cell-binding domain or a nucleic acid, vector or cell encoding the targeting module,
wherein the tag-binding domain of the targeting module binds to the tag of the switchable chimeric antigen receptor or the tag of the targeting module binds to the tag-binding domain of the switchable chimeric antigen receptor.

As used herein, the term "CRISPR" (Clustered Regularly Interspaced Short Palindromic Repeats) refers to a system evolved in bacteria as an adaptive immune system to defend against viral attack. Upon exposure to a virus, short segments of viral DNA are integrated into the CRISPR locus of the bacterial genome. RNA is transcribed from a portion of the CRISPR locus that includes the viral sequence. That RNA, which contains sequence complimentary to the viral genome, mediates targeting of a Cas9 protein to the sequence in the viral genome. The Cas9 protein cleaves and thereby silences the viral target. The CRISPR/Cas system has been adapted for genome editing in eukaryotic cells. The introduction of site-specific single (SSBs) or double strand breaks (DSBs) allows for target sequence alteration through, for example, non-homologous end-joining (NHEJ) or homology-directed repair (HDR).

In embodiments, the CRISPR system comprises an endonuclease and lipid nanoparticles comprising CRISPR-associated protein 9 mRNA and a target site-specific single guide RNA.

In embodiments, the target site-specific single guide RNA that causes downregulation of gene expression of an endogenous T cell receptor alpha chain comprises a nucleotide sequence according to SEQ ID NO. 1.

In embodiments, the target site-specific single guide RNA that causes downregulation of gene expression of HLA-A comprises a nucleotide sequence according to SEQ ID NO. 2.

In embodiments, the target site-specific single guide RNA that causes downregulation of gene expression of CIITA comprises a nucleotide sequence according to SEQ ID NO. 3.

**Table 1. Exemplary guide RNAs**

| SEQ ID No. to the Guide Sequence | Guide Sequence (SEQ ID No. 41 to 43) | Exemplary Full Sequence (SEQ ID No. 44 to 46) | Exemplary Mod. Sequence (SEQ ID No. 1 to 3) | Genomic Coordinates |
|---|---|---|---|---|
| SEQ ID NO. 1 | CUCUCAGCU GGUACACGGC A | CUCUCAGCUG GUACACGGCA GUUUUAGAGC UAGAAAUAGC AAGUUAAAAU AAGGCUAGUC CGUUAUCAAC UUGAAAAAGU GGCACCGAGU CGGUGCUUUU | mC*mU*mC*UC AGCUGGUACA CGGCAGUUUU AGAmGmCmU mAmGmAmAm AmUmAmGmC AAGUUAAAAU AAGGCUAGUC CGUUAUCAmA mCmUmUmGm AmAmAmAmAm GmUmGmGmC mAmCmCmGm AmGmUmCmG mGmUmGmCm U*mU*mU*mU | chr14:22547524 -22547544 |
| SEQ ID NO. 2 | ACAGCGACGC CGCGAGCCAG | ACAGCGACGC CGCGAGCCAG GUUUUAGAGC | mA*mC*mA*GC GACGCCGCGA GCCAGGUUUU | chr6:29942864-29942884 |
| | | UAGAAAUAGC AAGUUAAAAU AAGGCUAGUC CGUUAUCAAC UUGAAAAAGU GGCACCGAGU CGGUGCUUUU | AGAmGmCmU mAmGmAmAm AmUmAmGmC AAGUUAAAU AAGGCUAGUC CGUUAUCAmA mCmUmUmGm AmAmAmAmAm GmUmGmGmC mAmCmCmGm AmGmUmCmG mGmUmGmCm U*mU*mU*mU | |
| SEQ ID NO. 3 | CCCCCGGACG GUUCAAGCAA | CCCCCGGACG GUUCAAGCAA GUUUUAGAGC UAGAAAUAGC AAGUUAAAAU AAGGCUAGUC CGUUAUCAAC UUGAAAAAGU GGCACCGAGU CGGUGCUUUU | mC*mC*mC*CC GGACGGUUCA AGCAAGUUUU AGAmGmCmU mAmGmAmAm AmUmAmGmC AAGUUAAAAU AAGGCUAGUC CGUUAUCAmA mCmUmUmGm AmAmAmAmAm GmUmGmGmC mAmCmCmGm AmGmUmCmG mGmUmGmCm U*mU*mU*mU | chr16:10906853 -10906873 |

Another aspect of the invention is a method for generating an engineered human T cell expressing a switchable chimeric antigen receptor, the method comprising:
(1) providing a human T cell,
(2) introducing into a human T cell a CRISPR system comprising a nucleic acid that causes downregulation of gene expression of an endogenous T cell receptor alpha chain in the T cell by transfection,
(3) introducing into the human T cell a CRISPR system comprising a nucleic acid that causes downregulation of gene expression of HLA-A in the T cell by transfection,
(4) introducing into the human T cell a CRISPR system comprising a nucleic acid that causes downregulation of gene expression of CIITA in the T cell by transfection, thereby generating the CRISPR-modified T cell, and
(5) introducing into to the human T cell a nucleic acid or a vector comprising a nucleic acid encoding a switchable chimeric antigen cell surface receptor that comprises:
   - a tag-binding domain or a tag,
   - an extracellular hinge and a transmembrane domain, and
   - a signal transduction domain,
   by transduction.

In preferred embodiments, the switchable chimeric antigen cell surface receptor is a reversed universal chimeric antigen cell surface receptor comprising a tag, an extracellular hinge and a transmembrane domain, and signal transduction domain.

In embodiments, the CRISPR system comprises a lipid nucleic acid assembly composition comprising an endonuclease or an mRNA encoding an endonuclease and a target site-specific single guide RNA.

In embodiments, the lipid nucleic acid assembly composition comprises lipid nanoparticles. In preferred embodiments, the lipid nucleic acid assembly composition is a lipid nanoparticle (LNP).

In embodiments, the lipid nanoparticles are a mixture of cationic and/or ionizable lipids and helper lipids.

In embodiments, the lipid nanoparticles are a mixture of an ionizable lipid, a helper lipid and PEG-DMG.

In embodiments, the endonuclease is CRISPR-associated enzyme 9.

In embodiments, the endonuclease is *S. pyogenes* Cas9, *N. meningitidis* Cas9, *S. thermophilus* Cas9 or *S. aureus* Cas9.

According to the invention, the method comprises the introduction of a CRISPR system comprising a nucleic acid that causes downregulation or elimination of gene expression of TRAC in the T cell by transfection.

In embodiments, the genetic modification in the TRAC gene comprises at least one nucleotide within the genomic coordinates chr14:22547524-chr14:22547544. In embodiments, the genetic modification in the TRAC gene comprises at least 10 or at least 15 contiguous nucleotides within the genomic coordinates.

In embodiments, the genetic modification in the TRAC gene comprises at least one nucleotide of an exon of the TRAC gene.

In embodiments, the genetic modification in the TRAC gene comprises at least one insertion, deletion, substitution, or deamination of at least one nucleotide within the genomic coordinates.

In embodiments, the genetic modification in the TRAC gene comprises an indel.

In embodiments, the TRAC gene expression is reduced or eliminated by a gene editing system that binds to a TRAC genomic target sequence comprising at least 5 contiguous nucleotides within the genomic coordinates, preferably within the genomic coordinates chr14:22547524 chr14:22547544.

In embodiments, the target site-specific single guide RNA in step (2) comprises a nucleotide sequence according to SEQ ID NO. 1 or a sequence that is at least 90%, preferably 95%, more preferably 99%; identical to the sequence according to SEQ ID NO. 1.

According to the invention, the method comprises the introduction of a CRISPR system comprising a nucleic acid that causes downregulation of gene expression of HLA-A in the T cell by transfection.

In embodiments, the genetic modification in the HLA-A gene comprises at least one nucleotide within the genomic coordinates chosen from: chr6:29942854-chr6:29942913 and chr6:29943518-chr6:29943619.

In embodiments, the T cell is homozygous for HLA-B and/or homozygous for HLA-C genotypes. Preferably, the T cell is homozygous for HLA-B and HLA-C genotypes.

In embodiments, the T cell has reduced or eliminated expression of at least one HLA-A allele selected from: HLA-A1, HLA-A2, HLA-A3, HLA-A11, and HLA-A24.

In embodiments, the genetic modification in the HLA-A gene comprises at least one nucleotide within the genomic coordinates chr6:29942864-chr6:29942903, preferably chr6:29942876-chr6:29942897.

In embodiments, the genetic modification in the HLA-A gene comprises at least one nucleotide within the genomic coordinates chr6:29943528-chr6:29943609, preferably chr6:29943528-chr6:29943550.

In embodiments, the genetic modification in the HLA-A gene comprises at least 5, 6, 7, 8, 9, or 10 contiguous nucleotides within the genomic coordinates, preferably at least 10, at least 15, at least 16, at least 17, at least 18, at least 19, or at least 20 contiguous nucleotides within the genomic coordinates.

In embodiments, the genetic modification in the HLA-A gene comprises at least one C to T substitution or at least one A to G substitution within the genomic coordinates.

In embodiments, the genetic modification in the HLA-A gene comprises an indel.

In embodiments, the HLA-A expression is reduced or eliminated by a gene editing system that binds to an HLA-A genomic target sequence comprising at least 5 contiguous nucleotides within the genomic coordinates, preferably within the genomic coordinates chosen from: chr6:29942864-29942884; chr6:29942868-29942888; chr6:29942876-29942896; chr6:29942877-29942897; chr6:29942883-29942903; chr6:29943126-29943146; chr6:29943528-29943548; chr6:29943529-29943549; chr6:29943530-29943550; chr6:29943537-29943557; chr6:29943549-29943569; chr6:29943589-29943609; and chr6:29944026-29944046.

In embodiments, HLA-B allele is selected from any one of the following HLA-B alleles: HLA-B*07:02; HLA-B*08:01; HLA-B*44:02; HLA-B*35:01; HLA-B*40:01; HLA-B*57:01; HLA-B*14:02; HLA-B*15:01; HLA-B*13:02; HLA-B*44:03; HLA-B*38:01; HLA-B*18:01; HLA-B*44:03; HLA-B*51:01; HLA-B*49:01; HLA-B*15:01; HLA-B*18:01; HLA-B*27:05; HLA-B*35:03; HLA-B*18:01; HLA-B*52:01; HLA-B*51:01; HLA-B*37:01; HLA-B*53:01; HLA-B*55:01; HLA-B*44:02; HLA-B*44:03; HLA-B*35:02; HLA-B*15:01; and HLA-B*40:02.

In embodiments, the HLA-C allele is selected from any one of the following HLA-C alleles: HLA-C*07:02; HLA-C*07:01; HLA-C*05:01; HLA-C*04:01 HLA-C*03:04; HLA-C*06:02; HLA-C*08:02; HLA-C*03:03; HLA-C*06:02; HLA-C*16:01; HLA-C*12:03; HLA-C*07:01; HLA-C*04:01; HLA-C*15:02; HLA-C*07:01; HLA-C*03:04; HLA-C*12:03; HLA-C*02:02; HLA-C*04:01; HLA-C*05:01; HLA-C*12:02; HLA-C*14:02; HLA-C*06:02; HLA-C*04:01; HLA-C*03:03; HLA-C*07:04; HLA-C*07:01; HLA-C*04:01; HLA-C*04:01; and HLA-C*02:02.

In embodiments, HLA-B allele is selected from any one of the following HLA-B alleles: HLA-B*07:02; HLA-B*08:01; HLA-B*44:02; HLA-B*35:01; HLA-B*40:01; HLA-B*57:01; HLA-B*14:02; HLA-B*15:01; HLA-B*13:02; HLA-B*44:03; HLA-B*38:01; HLA-B*18:01; HLA-B*44:03; HLA-B*51:01; HLA-B*49:01; HLA-B*15:01; HLA-B*18:01; HLA-B*27:05; HLA-B*35:03; HLA-B*18:01; HLA-B*52:01; HLA-B*51:01; HLA-B*37:01; HLA-B*53:01; HLA-B*55:01; HLA-B*44:02; HLA-B*44:03; HLA-B*35:02; HLA-B*15:01; and HLA-B*40:02; and the HLA-C allele is selected from anyone of the following HLA-C alleles: HLA-C*07:02; HLA-C*07:01; HLA-C*05:01; HLA-C*04:01 HLA-C*03:04; HLA-C*06:02; HLA-C*08:02; HLA-C*03:03; HLA-C*06:02; HLA-C*16:01; HLA-C*12:03; HLA-C*07:01; HLA-C*04:01; HLA-C*15:02; HLA-C*07:01; HLA-C*03:04; HLA-C*12:03; HLA-C*02:02; HLA-C*04:01; HLA-C*05:01; HLA-C*12:02; HLA-C*14:02; HLA-C*06:02; HLA-C*04:01; HLA-C*03:03; HLA-C*07:04; HLA-C*07:01; HLA-C*04:01; HLA-C*04:01; and HLA-C*02:02.

In preferred embodiments, the HLA-B and HLA-C alleles are selected from any one of the following HLA-B and HLA-C alleles: HLA-B*07:02 and HLA-C*07:02; HLA-B*08:01 and HLA-C*07:01; HLA-B*44:02 and HLA-C*05:01; HLA-B*35:01 and HLA-C*04:01; HLA-B*40:01 and HLA-C*03:04; HLA-B*57:01 and HLA-C*06:02; HLA-B*14:02 and HLA-C*08:02; HLA-B*15:01 and HLA-C*03:03; HLA-B*13:02 and HLA-C*06:02; HLA-B*44:03 and HLA-C*16:01; HLA-B*38:01 and HLA-C*12:03; HLA-B*18:01 and HLA-C*07:01; HLA-B*44:03 and HLA-C*04:01; HLA-B*51:01 and HLA-C*15:02; HLA-B*49:01 and HLA-C*07:01; HLA-B*15:01 and HLA-C*03:04; HLA-B*18:01 and HLA-C*12:03; HLA-B*27:05 and HLA-C*02:02; HLA-B*35:03 and HLA-C*04:01; HLA-B*18:01 and HLA-C*05:01; HLA-B*52:01 and HLA-C*12:02; HLA-B*51:01 and HLA-C*14:02; HLA-B*37:01 and HLA-C*06:02; HLA-B*53:01 and HLA-C*04:01; HLA-B*55:01 and HLA-C*03:03; HLA-B*44:02 and HLA-C*07:04; HLA-B*44:03 and HLA-C*07:01; HLA-B*35:02 and HLA-C*04:01; HLA-B*15:01 and HLA-C*04:01; and HLA-B*40:02 and HLA-C*02:02.

In embodiments, the T-cell further comprises a reduced or eliminated surface expression of HLA-B relative to an unmodified T cell by a genetic modification in the HLA-B gene and/or a reduced or eliminated surface expression of HLA-C relative to an unmodified T cell by a genetic modification in the HLA-C gene.

In further embodiments, the T-cell further comprises a reduced or eliminated surface expression of HLA-B relative to an unmodified T cell by a genetic modification in the HLA-B gene and the T cell is homozygous for HLA-C genotype.

In alternative embodiments, the T-cell further comprises a reduced or eliminated surface expression of HLA-C relative to an unmodified T cell by a genetic modification in the HLA-C gene and the T cell is homozygous for HLA-B genotype.

In further alternative embodiments, the T-cell further comprises a reduced or eliminated surface expression of HLA-B relative to an unmodified T cell by a genetic modification in the HLA-B gene and a reduced or eliminated surface expression of HLA-C relative to an unmodified T cell by a genetic modification in the HLA-C gene.

In embodiments, the target site-specific single guide RNA in step (3) comprises a nucleotide sequence according to SEQ ID NO. 2 or a sequence that is at least 90%, preferably 95%, more preferably 99%; identical to the sequence according to SEQ ID NO. 2.

In embodiments, the HLA-A guide RNA comprises at least one modification, preferably selected from the group comprising a 2'-O-methyl (2'-O-Me) modified nucleotide, a phosphorothioate (PS) bond between nucleotides, a 2'-fluoro (2'-F) modified nucleotide, a modification at one or more of the first five nucleotides at the 5' end of the guide RNA, a modification at one or more of the last five nucleotides at the 3' end of the guide RNA, a PS bond between the first four nucleotides of the guide RNA, a PS bond between the last four nucleotides of the guide RNA, a 2'-O-Me modified nucleotide at the first three nucleotides at the 5' end of the guide RNA, a 2'-O-Me modified nucleotide at the last three nucleotides at the 3' end of the guide RNA, or combinations thereof.

In embodiments, the method further comprises matching the engineered human T cell for the HLA-B and HLA-C alleles to a patient.

In alternative embodiments, the method further comprises introducing into the human T cell a CRISPR system comprising a nucleic acid that causes downregulation of gene expression of HLA-B in the T cell and/or introducing into the human T cell a CRISPR system comprising a nucleic acid that causes downregulation of gene expression of HLA-C in the T cell by transfection.

According to the invention, the method comprises the introduction of a CRISPR system comprising a nucleic acid that causes downregulation of gene expression of CIITA in the T cell by transfection.

In embodiments, the genetic modification in the CIITA gene comprises at least one nucleotide of a splice site within the genomic coordinates chr16:10902171-chr16:10923242.

In embodiments, the genetic modification in the CIITA gene comprises a modification of at least one nucleotide of a splice acceptor site, preferably wherein the one nucleotide is A or G or T.

In embodiments, the genetic modification in the CIITA gene comprises a modification of a splice site boundary nucleotide.

In embodiments, the genetic modification in the CIITA gene comprises at least 5, 6, 7, 8, 9, or 10 contiguous nucleotides within the genomic coordinates chr16:10902171- chr16:10923242.

In embodiments, the genetic modification in the CIITA gene comprises at least one C to T substitution or at least one A to G substitution within the genomic coordinates chr16:10902171-chr16:10923242.

In embodiments, the genetic modification in the CIITA gene comprises an indel, a C to T substitution, or an A to G substitution within the genomic coordinates chosen from: chr16:10895410-10895430, chr16:10898649-10898669, chr16:10898658-10898678, chr16:10902171-10902191, chr16:10902173-10902193, chr16:10902174-10902194, chr16:10902179-10902199, chr16:10902183-10902203, chr16:10902184-10902204, chr16:10902644-10902664, chr16:10902779-10902799, chr16:10902788-10902808, chr16:10902789-10902809, chr16:10902790-10902810, chr16:10902795-10902815, chr16:10902799-10902819, chr16:10903708-10903728, chr16:10903713-10903733, chr16:10903718-10903738, chr16:10903721-10903741, chr16:10903723-10903743, chr16:10903724-10903744, chr16:10903873-10903893, chr16:10903878-10903898, chr16:10903905-10903925, chr16:10903906-10903926, chr16:10904736-10904756, chr16:10904790-10904810, chr16:10904811-10904831, chr16:10906481-10906501, chr16:10906485-10906505, chr16:10906486-10906506, chr16:10906487-10906507, chr16:10906492-10906512, chr16:10908127-10908147, chr16:10908130-10908150, chr16:10908131-10908151, chr16:10908132-10908152, chr16:10908137-10908157, chr16:10908138-10908158, chr16:10908139-10908159, chr16:10909006-10909026, chr16:10909007-10909027, chr16:10909018-10909038, chr16:10909021-10909041, chr16:10909022-10909042, chr16:10909172-10909192, chr16:10910165-10910185, chr16:10910176-10910196, chr16:10910186-10910206, chr16:10915547-10915567, chr16:10915551-10915571, chr16:10915552-10915572, chr16:10915567-10915587, chr16:10916348-10916368, chr16:10916359-10916379, chr16:10916362-10916382, chr16:10916449-10916469, chr16:10916450-10916470, chr16:10916455-10916475, chr16:10916456-10916476, chr16:10918423-10918443, chr16:10918504-10918524, chr16:10918511-10918531, chr16:10918512-10918532, chr16:10918539-10918559, chr16:10922153-10922173, chr16:10922478-10922498, chr16:10922487-10922507, chr16:10922499-10922519, chr16:10923205-10923225, chr16:10923214-10923234, chr16:10923218-10923238, chr16:10923219-10923239, chr16:10923220-10923240, chr16:10923221-10923241, and chr16:10923222-10923242.

In embodiments, the genetic modification in the CIITA gene comprises at least 5 contiguous nucleotides within the genomic coordinates chosen from: chr16:10906485-10906505, chr16:10906486-10906506, chr16:10906487-10906507, chr16:10906492-10906512, chr16:10908127-10908147, chr16:10908130-10908150, chr16:1090813I-10908151, chr16:10908132-10908152, chr16:10908137-10908157, chr16:10908138-10908158, chr16:10908139-10908159, chr16:10909006-10909026, chr16:10909007-10909027, chr16:10909018-10909038, chr16:10909021-10909041, chr16:10909022-10909042, chr16:10909172-10909192, chr16:10910165-10910185, chr16:10910176-10910196, chr16:10910186-10910206, chr16:10915547-10915567, chr16:10915551-10915571, chr16:10915552-10915572, chr16:10915567-10915587, chr16:10916348-10916368, chr16:10916359-10916379, chr16:10916362-10916382, chr16:10916449-10916469, chr16:10916450-10916470, chr16:10916455-10916475, chr16:10916456-10916476, chr16:10918423-109I8443, chr16:10918504-10918524, chr16:10918511-10918531, chr16:10918512-10918532, chr16:10918539-10918559, chr16:10922153-10922173, chr16:10922478-10922498, chr16:10922487-10922507, chr16:10922499-10922519, chr16:10923205-10923225, chr16:10923214-10923234, chr16:10923218-10923238, chr16:10923219-10923239, chr16:10923220-10923240, chr16:10923221-10923241, and chr16:10923222-10923242, preferably chr16:10908132-10908152, chr16:10908131-10908151, chr16:10916456-10916476, chr16:10918504-10918524, chr16:10909022-10909042, chr16:10918512-10918532, chr16:10918511-10918531, chr16:10895742-10895762, chr16:10916362-10916382, chr16:10916455-10916475, chr16:10909172-10909192, chr16:10906492-10906512, chr16:10909006-10909026, chr16:10922478-10922498, chr16:10895747-10895767, chr16:10916348-10916368, chr16:10910186-10910206, chr16:10906481-10906501, chr16:10909007-10909027, chr16:10895410-10895430, and chr16:10908130-10908150.

In embodiments, the genetic modification in the CIITA gene comprises at least 10 or at least 15 contiguous nucleotides within the genomic coordinates.

In embodiments, target site-specific single guide RNA in step (4) is a CIITA genomic target sequence that comprises at least one nucleotide of a splice acceptor site or a splice donor site.

In preferred embodiments, the one nucleotide is the splice site boundary nucleotide at the splice acceptor site or the splice site boundary nucleotide at the splice donor site.

In embodiments, target site-specific single guide RNA in step (4) comprises a guide sequence that directs an RNA-guided DNA binding agent to make a cut in a CIITA genomic target sequence that is 4 nucleotides or less, is 3 nucleotides or less, is 2 nucleotides or less, or is 1 nucleotide from a splice site boundary nucleotide.

In embodiments, the target site-specific single guide RNA in step (4) comprises a nucleotide sequence according to SEQ ID NO. 3 or a sequence that is at least 90%, preferably 95%, more preferably 99%; identical to the sequence according to SEQ ID NO. 3.

In embodiments, the CIITA guide RNA comprises at least one modification, preferably selected from the group comprising a 2'-O-methyl (2'-O-Me) modified nucleotide, a phosphorothioate (PS) bond between nucleotides, a 2'-fluoro (2'-F) modified nucleotide, a modification at one or more of the first five nucleotides at the 5' end of the guide RNA, a modification at one or more of the last five nucleotides at the 3' end of the guide RNA, a PS bond between the first four nucleotides of the guide RNA, a PS bond between the last four nucleotides of the guide RNA, a 2'-O-Me modified nucleotide at the first three nucleotides at the 5' end of the guide RNA, a 2'-O-Me modified nucleotide at the last three nucleotides at the 3' end of the guide RNA, or combinations thereof.

In embodiments, the genetic modification in the CIITA gene comprises an indel.

In embodiments, the introduction of a nucleic acid or a vector comprising a nucleic acid encoding a switchable chimeric antigen cell surface receptor according to step (5) is carried out by adding a non-viral or a viral vector compromising, but not limited to, lentiviral or adenoviral vectors. The introduction of a nucleic acid or a vector comprising a nucleic acid encoding a switchable chimeric antigen cell surface receptor according to step (5) may occur at a random site or in a site-directed manner including but not limited to homology-directed repair (HDR) of a CRISPR edited locus.

In preferred embodiments, the introduction of a nucleic acid or a vector comprising a nucleic acid encoding a switchable chimeric antigen cell surface receptor according to step (5) is carried out by adding self-inactivating retroviral vector particles.

A further aspect of the invention is a method for stimulating a chimeric antigen receptor-mediated immune response in a mammal, preferably a human, having cancer, an infectious or an autoimmune disease by administration of an engineered human T cell according to the invention and a targeting module, a pharmaceutical composition or a kit according to the invention, preferably to a subject in need thereof.

For therapeutic applications, a sterile pharmaceutical composition according to the invention or a sterile kit according to the invention, comprising a pharmacologically effective quantity of the engineered human T cell according to the invention and a targeting module, is administered to a subject in order to treat the aforementioned illnesses.

In some embodiments, the method for stimulating a chimeric antigen receptor-mediated immune response in a mammal; preferably, the method for treatment of cancer, infectious or autoimmune disease, comprises the following steps:
a) administering to a mammal an effective amount of a targeting module and
b) administering to the mammal an effective amount of an engineered human T cell according to the invention,
   wherein the tag-binding domain of the targeting module binds to the tag of the switchable chimeric antigen receptor or the tag of the targeting module binds to the tag-binding domain of the switchable chimeric antigen receptor,
wherein the targeting module is administered to a mammal prior to, concurrent with or after the administration of the vector or cell.

In embodiments, the method for stimulating a chimeric antigen receptor-mediated immune response in a mammal further comprises determining the HLA-B and HLA-C alleles of the recipient subject (patient) and matching the engineered human T cell for the HLA-B and HLA-C alleles to a patient.

In embodiments, the patient comprises the HLA-B and HLA-C alleles of the engineered human T cell.

In alternative embodiments, the patient comprises one or more HLA-B and HLA-C alleles of the engineered human T cell.

In further embodiments, the method for stimulating a chimeric antigen receptor-mediated immune response in a mammal; preferably, the method for treatment of cancer, infectious or autoimmune disease, comprises the following steps:
a) administering to a mammal an effective amount of a targeting module comprising at least one target cell-binding domain and a tag-binding domain or a tag,
   wherein the at least one target cell-binding domain is an antibody, antibody fragment, a protein, a peptide or a low molecular weight organic ligand that binds to surface antigens selected from the group comprising CD2, CD3, CD4, CD5, CD7, CD8, CD10, CD15, CD19, CD20, CD22, CD23, CD25, CD30, CD33, CD38, CD44, CD44v6 CD52, CD66a, CD66b, CD66c, CD66d, CD66e, CD66f, CD90, CD99, CD123, CD133, CD135, CD150 CD181, CD182, CD184, CD223, CD229, CD269, CD273, CD274, CD276, CD279, CD319, CD366 and CD371, cytokine receptors, preferably interleukin receptors, especially preferred IL-8Rα, IL-8Rβ, IL-11Rα, IL-11Rβ, IL13Rα1; CXCR4, c-Met, mesothelin, members of the epidermal growth factor receptor family and mutants thereof, especially preferred ErbB1, ErbB2, ErbB3, ErbB4 or mutants thereof; members of the tumor necrosis factor receptor superfamily, claudins, ephrins, ephrin receptors, especially preferred EphA1-10, EphA5 or EphB1-6; fucosyl transferases, prostate specific antigens, preferably PSCA and PSMA; embryonic antigens, preferably CEA and fetal acethylcholine receptor; members of the vascular endothelia growth factor family, EpCAM, AFP, members of the intercellular adhesion molecule family, C-type lectins, integrins, members of the mucin protein family, FSHR, HMW-MAA, FBP, folate receptors, somatostatin receptors, ligands of the NKG2D receptor, members of the epithelia glycoprotein family, diasialogangliosides, glypicans, G protein-coupled receptors, human papillomavirus proteins, cancer-testis antigens, fibroblast activation proteins, members of the carbonic anhydrase family, members of the carbohydrate antigen family, Notch ligands, MCSP, glycoprotein A33, guanylate cyclase 2C and tumor-specific glycans, including mutants and analogues of the named antibodies, antibody fragments, proteins, peptides or low molecular weight organic ligands, and
b) administering to the mammal an effective amount of an engineered human T cell, and
c) administering to a mammal an effective amount of a further targeting module,
wherein the targeting module and the at least one further targeting module comprise different target cell-binding domains, and identical tag-binding domains or tags, wherein the method is carried out in the order of the steps a), b) and c).

In preferred embodiments, the targeting module is administered on its own, preferably one hour to 2 days, more preferably 4 to 24 hours, prior to the administration of the engineered human T cell and the targeting module is administered until, preferably in the range of 3 days to 30 days, after the administration of the engineered human T cell. Furthermore, additional such doses of the targeting module may be administered following resting periods to reactivate the switchable chimeric antigen receptor-carrying effector cells.

The invention is not limited to the embodiments shown and described, but also includes all embodiments having the same effect within the meaning of the invention. Furthermore, the invention is also not limited to the specifically described combinations of features but may also be defined by any other combination of specific features of all the individual features disclosed as a whole, provided that the individual features are not mutually exclusive, or a specific combination of individual features is not explicitly excluded.

In the following, the invention will be explained in more detail by means of an embodiment example. The embodiment example refers to the engineered human T cell and the method for generating it and is intended to describe the invention without limiting it.

Implementations of the invention will be described, by way of example only, with reference to accompanying drawings in which:
**Fig. 1** shows a schema of a switchable chimeric antigen receptor (CAR) with three domains, wherein the first domain is a tag-binding domain or a tag (exemplified as scFv), the second domain is an extracellular hinge and a transmembrane domain, and the third domain is a signal transduction domain, and the optional fourth domain is a short peptide linker in the extracellular portion of the receptor.
**Fig. 2** shows a schematic illustration of the mode of action of engineered T cell according to the invention in combination with an antigen-specific targeting module (TM) together forming the active drug. A RevCAR expressing allogeneic T cell (Allo-RevCAR-T) carries the RevCAR epitope (RCE or tag), preferably a short, non-immunogenic peptide motif derived from the human nuclear La/SSB autoantigen, on their cell surface. Since ligands binding to RCE or tag are not present within the human body Allo-RevCAR-T remain in an off-mode (left). Antigen-specificity of Allo-RevCAR-T is provided via a soluble targeting module (TM) with exclusive specificity for a target antigen. The TM for Allo-RevCAR consists of a target-binding domain and a tag-binding domain specific for the RCE or tag. Cross-linking of RevCAR-T and the target antigen-expressing tumor cell by TMs activates RevCAR-T effector functions and subsequently killing of the tumor cells (right).

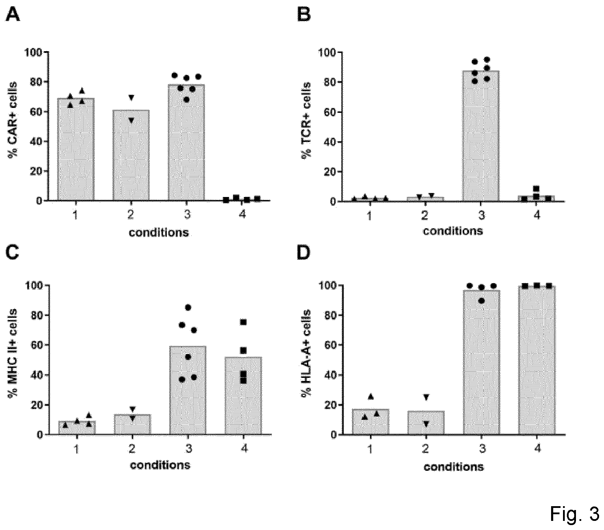

**Fig. 3** shows the transduction efficacy, and TRAC, CIITA, and HLA-A knockout frequencies of Allo-switchable CAR-T cells. Frequency of switchable CAR expression (A), frequency of TCR knock-out (B), MHC class II (C), and HLA-A knock-out (D) are shown for the following experimental conditions: (1) LNP-mediated edits in the sequence TRAC - CIITA - HLA-A combined with retroviral vector (RV) mediated gene transfer; (2) LNP edits in the sequence TRAC - HLA-A - CIITA combined with RV-mediated gene transfer; and for control (3) only RV-mediated gene transfer, and (4) only LNP-mediated edit of the TRAC locus. The order of edits may be varied. Each dot represents an individual donor.

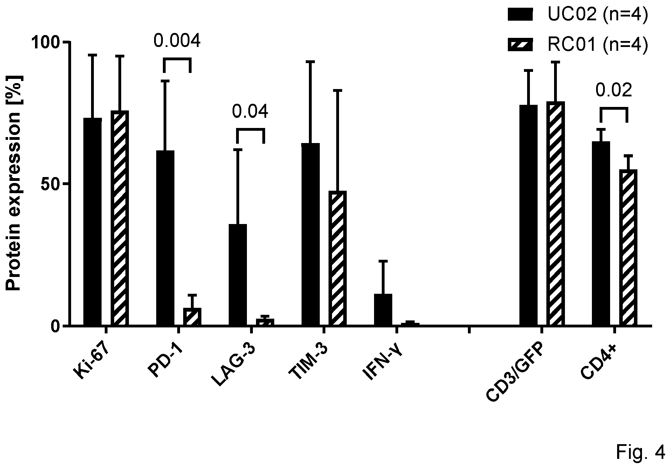

**Fig. 4** shows a comparison of the protein expression of proliferation marker Ki-67, exhaustion markers PD-1, LAG-3, TIM-3, IFN-γ and additional phenotypic markers of human primary T cells transduced to express a switchable CAR (UniCAR or RevCAR). Unpaired t-test was applied for statistical analysis, and p-values below 0.05 are shown on top of each plot.

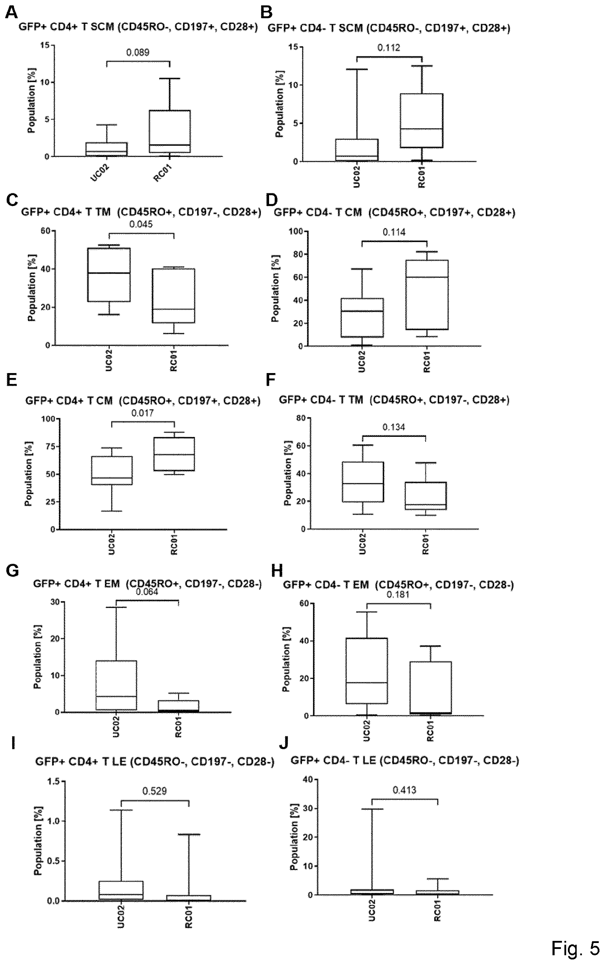

**Fig. 5** shows a comparison of the differentiated phenotype following production of human primary T cells transduced to express a switchable CAR (UniCAR = UC02: n=10 or RevCAR = RC01: n=9). T cells were stained for cell surface markers CD4, CD45RO, CD197 and CD28. Cells were gated on GFP+ and CD4+ (**A, C, E, G, I**) or CD4- (**B, D, F, H, J**) respectively and percentages of effector/memory populations were determined based on expression of CD45RO, CD197 and CD28. Unpaired t-test was applied for statistical analysis, and p-values are shown on top of each plot.

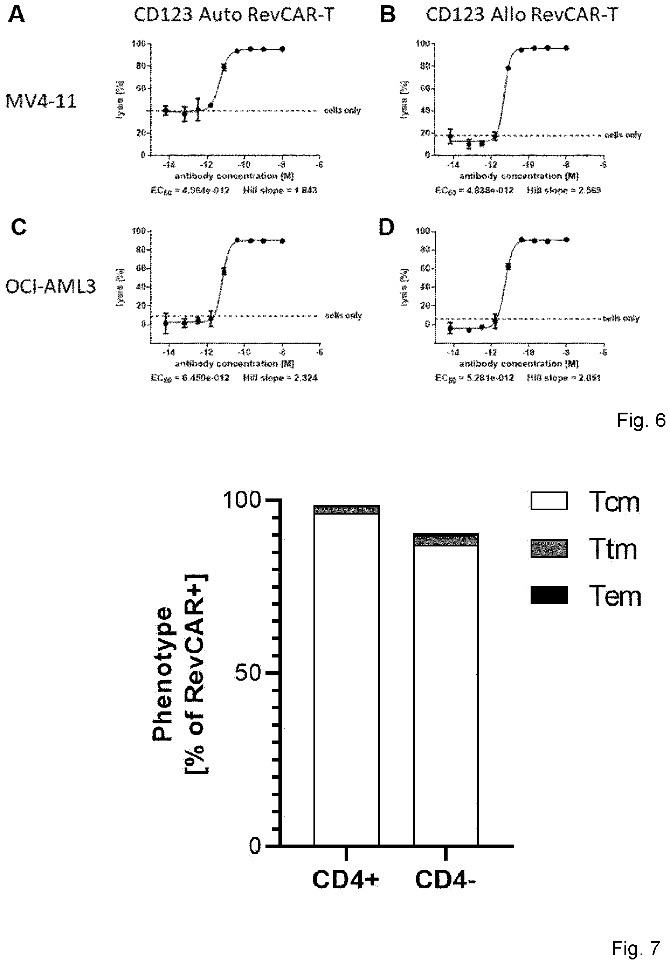

**Fig. 6** shows the cell lysis of CD123 expressing AML cell line MV4-11 (**A**, **B**) or OCI-AML3 (**C**, **D**) determined by flow cytometry after 48 h in the presence of human primary T cells expressing a switchable CAR (Auto RevCAR-T, **A, C**) or additionally edited via CRISPR/Cas mediated knock out of the TRAC locus (Allo RevCAR-T, **B**, **D**) and different concentrations of anti-CD 123 targeting module (R-TM123). The dotted line shows unspecific lysis in absence of R-TM123.
**Fig. 7** shows a RevCAR-T product manufactured using a clinical-scale, GMP-compliant process revealing a selective enrichment in CD28, and C-C chemokine receptor type 7 (CCR7, CD197) double-positive T cells classified as central-memory T cells (Tcm) according to the classification of human T cell subsets introduced by Mahnke and colleagues (Mahnke *et al.* 2013). Therefore, formulated, cryopreserved samples of the final drug product were thawed and stained for the surface markers CD4, CD28, CD45RA, CD45RO, and CD197 (CCR7). Human T cell subsets were classified according to Mahnke and colleagues (Mahnke *et al.* 2013). Tcm, central memory T cells CD28+ CD197+, Ttm, transitional memory T cells CD28+ CD197-; Tem, effector memory T cells CD28- CD197-.

### Gene editing

Gene editing is achieved by transfecting sgRNAs specific for the target genes alongside Cas9 mRNA or protein into the cells to be edited. Any method of transfection could be applied including electroporation and lipofection or lipid-mediated delivery.

### Guide Selection

CRISPR/Cas9 sgRNA target sequences are defined as the 20 nucleotides five prime (5) of an NGG protospacer adjacent motif (PAM). sgRNAs were selected for genomic loci targeting cut-sites within coding sequences of TRAC (ENSG00000277734), CIITA (ENSG00000179583) and HLA-A (ENSG00000277734). This excluded sgRNAs that would cut within introns or within 5' or 3' untranslated regions (UTRs). sgRNAs that were determined to overlap with high minor allele frequency (MAF) single nucleotide polymorphisms (SNPs) found in GenomAD were excluded (Lek *et al.* 2016). A selection of the top in silico predicted guides for TRAC, CIITA and HLA-A knockout were screened in primary T cells for on-target insertion/deletion (indel) quantification using targeted next-generation sequencing (NGS) of polymerase chain reaction (PCR) amplicons derived from the target sites, herein referred to as amplicon-seq. The editing percentage (e. g., the "editing efficiency" or "percent editing" or "indel frequency") is defined as the total number of sequencing reads with insertions/deletions ("indels") or substitutions over the total number of sequencing reads, including wild type. Nucleotides within ten base pairs of the intended cut site are evaluated for insertions, deletions, and mismatches to the reference sequence. Qualification of amplicon-seq for indel quantification using control guides has demonstrated a limit of detection as low as 0.5 %. LNPs containing the most efficient guides for each target were tested on primary T cells and knockout efficiency was quantified with NGS and with flow cytometry to confirm loss of protein expression (**Fig. 3**).

### Switchable CARs

For the genetical engineering to express switchable CARs, a polynucleotide vector encoding the switchable CAR and all necessary elements to ensure its expression in the genetically engineered immune cell is transferred into the immune cell. In particular, the switchable CAR comprises IL-2LP (modified human IL-2 leader peptide), RCE (RevCAR epitope, also tag), G4S1 (glycine-serine linker), ECD (extracellular domain), TMD (transmembrane domain), ICD (intracellular domain).

The transfer of the vector can be performed by electroporation or transfection of nucleic acids or the help of viral vector systems like adeno-, adeno-associated, retro-, foamy- or lentiviral viral gene transfer.

The lentiviral gene transfer is applied for stable expression of switchable CARs in immune cells by first constructing a lentiviral vector encoding for a selected switchable CAR. The lentiviral vector is pLVX-EF1alpha UniCAR 28/ζ (Clontech, Takara Bio Group), in which the lentiviral parts of the vector are derived from the human immunodeficiency virus (HIV) and the MSC/IRES/ZxGreenl portion was replaced by the switchable CAR construct.

The lentiviral particles are produced by transient transfection of human embryonal kidney (HEK) 293T (ACC 635) cells with the switchable CAR encoding lentiviral vector plasmid and cotransfection with a group specific antigen (gag) and Polymerase (pol) encoding plasmid (psPAX2) plus a plasmid encoding for an envelope (pMD2.G). After transfection, the packaging plasmid expresses Gag and Pol protein of HIV-1. The plasmid MD2.G encodes the glycoprotein of the vesicular stomatitis virus (VSV-G). VSV-G protein is used to lentiviral vectors to transduce a broad range of mammalian cells. Various envelopes from different virus species can be utilized for this purpose. Lentiviral vectors can successfully pseudotype with the envelope glycoproteins (Env) of amphotropic murine leukemia virus (MLV) or the G protein of vesicular stomatitis virus (VSV-G), a modified envelope of the prototypic foamy virus (PFV) or chimeric envelope glycoprotein variants derived from gibbon ape leukemia virus (GaLV) and MLV.

Supernatants from transfected HEK293T cells are harvested 24 h to 96 h after transfection and virus particles are concentrated from the supernatant by ultracentrifugation or other methods. For lentiviral transduction of immune cells, *peripheral blood mononuclear cells* (PBMC) or isolated T cells are activated with mab specific for the CD3 complex, e.g. clone OKT3 or UCHT1, either given in solution or coated to plastic cell culture dishes or magnetic beads or a biodegradable polymer matrix. Activation of PBMC or isolated T cells is further enhanced by stimulating costimulatory pathways with mabs or ligands specific for CD27, CD28, CD134 or CD137 either alone or in combinations coated to plastic cell culture dishes or magnetic beads or a biodegradable polymer matrix and the supply with exogenous recombinant cytokines like interleukin (IL)-2, IL-7, IL-12, IL-15 and IL-21. Concentrated or non-concentrated virus particles are added to PBMC or T cell cultures 24 h to 96 h after initial administration of activating CD3 specific antibodies and/or antibodies specific for costimulatory receptors CD27, CD28, CD134 or CD137 and/or recombinant cytokines as single or multiple doses. T cell electroporation, transduction and expansion may be performed in open cell culture systems by manual handling or in closed partially or fully automated systems.

Stable transduction of T cells may be determined by flow cytometry after staining with tag-containing molecules for surface expression of switchable CARs or mabs directed against a fourth domain of switchable CARs from day 3 onwards after the final administration of virus supernatant. Switchable CAR transduced T cells can be propagated *in vitro* by culturing them under the supply of recombinant cytokines and activating anti-CD3 mabs.

In case the switchable CAR harbors the optional fourth domain, a peptide sequence forming a linear epitope for a mab, immune cells genetically modified to express switchable CARs can be specifically propagated *in vitro* by coating a mab or antibody fragments thereof binding to the fourth switchable CAR domain to the surface of culture dishes or to beads of any kind or a biodegradable polymer matrix, which are added to the cell culture at a defined ratio. The binding of surface-coated mabs to the switchable CAR peptide domain induces cross-linkage of cell-surface expressed switchable CARs and formation of an immune synapse, which leads to the activation of signal pathways specifically triggered by the signal domain of the switchable CAR. Depending on the signal pathways induced, this may lead to enhance proliferation and sustained resistance against activation-induced cell death of the switchable CAR-carrying immune cells and therefore enrichment of switchable CAR genetically modified immune cells in a mixed population.

The optional fourth domain, a peptide sequence forming a linear epitope for a mab, can be further utilized to enrich and purify switchable CAR-expressing immune cells from mixed populations. Enrichment and purification are performed with the help of a mab or antibody fragment thereof binding to the fourth switchable CAR domain to either mark switchable CAR-expressing cells for cell sorting or to transiently link the switchable CAR expressing immune cell to small particles, which can be utilized for cell isolation. In one aspect, switchable CAR-engrafted immune cells are incubated with the mab recognizing the fourth domain. Next, magnetic beads are added, which are conjugated with antibodies or fragments thereof directed against the species- and isotype-specific heavy and light chains of the mab binding to the optional fourth domain. Thus, switchable CAR-expressing immune cells and magnetic beads are linked and are trapped and separated from other immune cells in a magnetic field.

### Production of the engineered human T cell

T cells are isolated from fresh leukapheresis products of HLA-B/C typed healthy human donors. Selected T cells are characterized regarding purity (CD3+ frequency) and content (viability, viable cell count). Knockout of the T cell receptor alpha chain (TRAC), HLA-A and class II major histocompatibility complex transactivator (CIITA) is achieved via lipid nanoparticles (LNPs) containing Cas9 mRNA and single guide RNA (sgRNA) for either HLA-A, CIITA or TRAC. These LNPs consist of a lipid mixture (ionizable lipid, helper lipid, cholesterol, and PEGylated lipid) and a cargo of mRNA coding for Cas9 and the single guide RNA targeting the gene of interest. The LNPs are added to the culture sequentially, which significantly reduces the risk of translocation. The T cell culture is genetically modified to express RevCAR by adding self-inactivating retroviral vector particles. After the gene engineering process, and the cultures are expanded for additional 5 to 6 days before harvest. TCRα/β+ cells may be depleted.

### Characterization of the engineered human T cell according to the invention

Editing efficiencies and RevCAR or UniCAR transduction efficiencies were assessed by flow cytometry (**Figure 3**). The functionality of the engineered human T cell described in this invention was assessed regarding the protein expression of proliferation marker, exhaustion markers and additional phenotypic markers and the phenotype following production of human primary T cells.

**Figure 4** shows a comparison of the protein expression of proliferation marker Ki-67, exhaustion markers PD-1, LAG-3, TIM-3, IFN-γ and additional phenotypic markers of human primary T cells transduced to express a switchable CAR (UniCAR or RevCAR). Human primary T cells from 4 healthy donors were transduced via lentiviral vector mediated gene transfer to express a switchable CAR (UniCAR = UC02 or RevCAR = RC01). After harvest, T cells were stained for proliferation marker Ki-67 and exhaustion markers PD-1, LAG-3, TIM-3, IFN-γ and additional phenotypic markers. GFP expression of CD3+ cells was used as a surrogate for CAR positivity, as the CAR cDNA was followed by 2pA site and GFP. T cells transduced with RC01 showed significantly reduced expression of exhaustion markers PD-1 and LAG-3 and numerically reduced intracellular IFN-γ expression.

**Figure 5** shows a comparison of the differentiated phenotype following production of human primary T cells transduced to express a switchable CAR (UniCAR or RevCAR). Human primary T cells from healthy donors were transduced via lentiviral vector mediated gene transfer to express a switchable CAR (UniCAR02 = UC02 or RevCAR01 = RC01). After harvest at day 14, T cells were stained for cell surface markers CD4, CD45RO, CD197 and CD28. Cells were gated on GFP+ and CD4+ (**A, C, E, G, I**) or CD4- (**B, D, F, H, J**) respectively and percentages of effector/memory populations were determined based on expression of CD45RO, CD197 and CD28. T cells transduced with RC01 showed an enrichment of undifferentiated memory populations (SCM: stem cell memory, CM: central memory) and a reduced proportion of more differentiated populations (TM: transitional memory, EM: effector memory, LE: late effector). Thus, RevCAR-T cells showed a less differentiated phenotype following production.

### Design of targeting modules for the kit according to the invention

The targeting module R-TM123 is a soluble, recombinant fusion protein comprising two antibody-derived binding domains. One selectively binds to the target antigen CD123, the other recognizes the RCE or tag presented on RevCAR expressing cells (epitope E5B9 from the human La protein). Thus, R-TM123 functions as a bridging module between Allo-RevCAR-T and a CD123-expressing target cancer cell (Fig. 2). The targeting module further comprises an 8x-histidine tag for detection and purification purposes at the C-terminus.

### Cytotoxicity assay

The potency of anti-CD123 TMs to induce tumor cell elimination by switchable CAR-T cells was tested on a flow-cytometry-based cytotoxicity assay with AML cell line MV4-11 or OCI-AML3 (Fig. 6). Human primary T cells from the same healthy donor were transduced via lentiviral vector mediated gene transfer to express a switchable CAR (Auto RevCAR-T) or additionally edited via CRISPR/Cas mediated knock out of the TRAC locus (Allo RevCAR-T). TMs potency was assessed on a flow-cytometry-based cytotoxicity assay. Switchable CAR-T cells were incubated with the target cells at a E:T ratio of 2:1 in the presence of various TM concentrations for 48 h. Target cells were quantified and lysis was calculated normalizing the cell count of each sample to a control sample where only tumor cells were plated. Data were fitted with a four-parameter model with a variable slope for sigmoidal curves. The calculated EC50 value can be interpreted as a representative value for the TM potency against these tumor cells.

**Figure 6** shows the cell lysis of CD123 expressing AML cell line MV4-11 or OCI-AML3 in the presence of human primary T cells expressing a switchable CAR (Auto RevCAR-T) or additionally edited via CRISPR/Cas mediated knock out of the TRAC locus (Allo RevCAR-T) and different concentrations of anti-CD123 targeting module (R-TM123). The modified T cells were incubated with CD123 expressing AML cell line MV4-11 or OCI-AML3 in the presence of different concentrations of R-TM123 (i.e. antibody concentration). OCI-AML3 cells were stained with cell dye eFluor670 beforehand. Number of living eFlour670 positive AML cells were determined by flow cytometry after 48 h. Lysis was calculated by normalizing number of living AML cells to a control with AML cells only. Derived dose-response curves, EC50 values and Hill slopes are shown. The unspecific lysis was determined in absence of R-TM123. Respective EC50 values for Auto RevCAR-T and Allo RevCAR-T cells in this short-term assay are comparable. Thus, the lysis activity of RevCAR-T cells was not influenced by the TRAC edit. Further, unspecific lysis against MV4-11 cells was abrogated by the TRAC edit in Allo RevCAR-T cells.

The efficacy of adoptive immunotherapy including CAR-T requires that transferred T cells persist in vivo. This is of particular interest for switchable CAR-T platforms like the RevCAR platform, as long-term persistence enables a re-activation after pausing the soluble adaptor administration. Certain surface marker combinations have been associated with clinical superior T cell phenotypes, which enriched in a clinical T cell product enhances anti-tumor efficacy and overall clinical outcome (Mahnke *et al.* 2013).

Figure 7 shows the phenotypical analysis of a RevCAR-T product manufactured using a clinical-scale, GMP-compliant process. Therefore, formulated, cryopreserved samples of the final drug product were thawed and stained for the surface markers CD4, CD28, CD45RA, CD45RO, and CD197 (CCR7). Human T cell subsets were classified according to Mahnke and colleagues (Mahnke *et al.* 2013): Tcm, central memory T cells CD28+ CD197+, Ttm, transitional memory T cells CD28+ CD197-, Tem, effector memory T cells CD28- CD197-. A selective enrichment of CD28, and C-C chemokine receptor type 7 (CCR7, CD197) double-positive T cells is observed, which can be classified as central-memory T cells (Tcm). Beside the pre-dominantly Tcm phenotype found in the product, transitional memory T cells (Ttm) and memory effector T cells (Tem) could be detected in the RevCAR product in much lower quantities (Fig. 7). Most T cells (> 90%) express the two markers CD45RO and CD45RA simultaneously on the surface, as is typical for T cells that are in the process of cell division (LaSalle and Hafler 1991). Cell products comprising higher CD28 positive and/or CCR7 positive T cell fractions have been shown to correlate with objective tumor responses in non-human primate models and patients (reviewed in Busch *et al.* 2016; Gattinoni *et al.* 2017).

### Cited non-patent literature

Bae S, Park J, Kim JS (2014) Cas-OFFinder: a fast and versatile algorithm that searches for potential off-target sites of Cas9 RNA-guided endonucleases, Bioinformatics 30, 1473-1475. Brudno JN, Kochenderfer JN (2016) Toxicities of chimeric antigen receptor T cells: recognition and management. Blood 127, 3321-3330.
Busch DH, Fräßle SP, Sommermeyer D, Buchholz VR, Riddell SR (2016) Role of memory T cell subsets for adoptive immunotherapy. Semin Immunol 28, 28-34.
Cameron P, Fuller CK, Donohoue PD, Jones BN, Thompson MS, Carter MM, Gradia S, Vidal B, Garner E, Slorach EM, Lau E, Banh LM, Lied AM, Edwards LS, Settle AH, Capurso D, Llaca V, Deschamps S, Cigan M, Young JK, May AP (2017) Mapping the genomic landscape of CRISPR-Cas9 cleavage. Nat Methods 14, 600-606.
Cartellieri M, Bachmann M, Feldmann A, Bippes C, Stamova S, Wehner R, Temme A, Schmitz M (2010) Chimeric Antigen Receptor-Engineered T Cells for Immunotherapy of Cancer J. Biomed. Biotechnol. Article ID 956304, doi: 10.1155/2010/956304.
Cartellieri M, Feldmann A, Koristka S, Arndt C, Loff S, Ehninger A, von Bonin M, Bejestani EP, Ehninger G, Bachmann MP (2016) Switching CAR T cells on and off: a novel modular platform for retargeting of T cells to AML blasts. Blood cancer J. 6 (8), e458.
Gattinoni L, Speiser DE, Lichterfeld M, Bonini C (2017) T memory stem cells in health and disease. Nature Medicine 23, 18-27.
Giannoukos G, Ciulla DM, Marco E, Abdulkerim HS, Barrera LA, Bothmer A, Dhanapal V, Gloskowski SW, Jayaram H, Maeder ML, Skor MN, Wang T, Myer VE, Wilson CJ (2018) UDiTaS™, a genome editing detection method for indels and genome rearrangements. BMC Genomics 19, 212.
Guedan S, Madar A, Casado-Medrano V, Shaw CE, Wing A, Liu F, Young RM, June CH, Posey AD (2020) Single residue in CD28-costimulated CAR T cells limits long-term persistence and antitumor durability. J Clin Invest. 133215.
LaSalle JM, Hafler DA (1991) The coexpression of CD45RA and CD45RO isoforms on T cells during the S/G2/M stages of cell cycle. Cellular immunology 138, 197-206.
Lek M, Karczewski KJ, Minikel EV, Samocha KE, Banks E, Fennell T, O'Donnell-Luria AH, Ware JS, Hill AJ, Cummings BB, Tukiainen T, Birnbaum DP, Kosmicki JA, Duncan LE, Estrada K, Zhao F, Zou J, Pierce-Hoffman E, Berghout J, Cooper DN, Deflaux N, DePristo M, Do R, Flannick J et al. (2016) Analysis of protein-coding genetic variation in 60,706 humans. Nature 536, 285-291. Mahnke YD, Brodie TM, Sallusto F, Roederer M, Lugli E (2013) The who's who of T-cell differentiation: human memory T-cell subsets. Eur J Immunol 43, 2797-2809.
Morgan RA, Yang JC, Kitano M, Dudley ME, Laurencot CM, Rosenberg SA (2010) Case Report of a Serious Adverse Event Following the Administration of T Cells Transduced With a Chimeric Antigen Receptor Recognizing ERBB2. Mol. Ther. 18, 843-851.
Neelapu SS, Munoz J, Locke FL, Miklos DB, Brown R, McDevitt JT, Mardiros A, Demirhan E, Konto C, Tees MT (2020) First-in-human data of ALLO-501 and ALLO-647 in relapsed/refractory large B-cell or follicular lymphoma (R/R LBCL/FL): ALPHA study. Journal of Clinical Oncology, 38: 8002-8002.
Pinello L, Canver MC, Hoban MD, Orkin SH, Kohn DB, Bauer DE, Yuan GC (2016) Analyzing CRISPR genome-editing experiments with CRISPResso. Nat Biotechnol 34, 695-697.
Reinke AW, Grant RA, Keating AE (2010) A Synthetic Coiled-Coil Interactome Provides Heterospecific Modules for Molecular Engineering. JACS 132, 6025-6031.
Sotillo E, Barrett DM, Black K., Bagashev A, Oldridge D, Wu G, Sussman R, Lanauze C, Ruella M, Gazzara MR, Martinez NM, Harrington CT, Chung EY, Perazzelli J, Hofmann TJ, Maude SL, Raman P, Barrera A, Gill S, Lacey SF, Melenhorst JJ, Allman D, Jacoby E, Fry T, Mackall C, Barash Y, Lynch KW, Maris JM, Grupp SA, Thomas-Tikhonenko A (2015) Convergence of Acquired Mutations and Alternative Splicing of CD19 Enables Resistance to CART-19 Immunotherapy. Cancer Discov. 5, 1282-1295.
Zhang Y (2021) A Novel Strategy for Off-the-shelf T Cell Therapies Evading Host T Cell and NK Cell Rejection. Oral presentation. European Society of Gene and Cell Therapy Annual Congress. Titov A, Petukhov A, Staliarova A, Motorin D, Bulatov E, Shuvalov O, Soond SM, Piacentini M, Melino G, Zaritskey A, Barlev NA (2018) The biological basis and clinical symptoms of CAR-T therapy-associated toxicites. Cell Death Dis 9, 897.
Weber EW, Parker KR, Sotillo E, Lynn RC, Anbunathan H, Lattin J, Good Z, Belk JA, Daniel B, Klysz D, Malipatlolla M, Xu P, Bashti M, Heitzeneder S, Labanieh L, Vandris P, Majzner RG, Qi Y, Sandor K, Chen LC, Prabhu S, Gentles AJ, Wandless TJ, Satpathy AT, Chang HY, Mackall CL (2021) Transient rest restores functionality in exhausted CAR-T cells through epigenetic remodeling. Science 372 (6537), eaba1786.

### List of reference signs

- 1: first domain, a tag-binding domain or tag
- 2: second domain, an extracellular hinge and a transmembrane domain
- 3: third domain, a signal transduction domain
- 4: optional fourth domain, a short peptide linker

## Claims

1. An engineered human T cell, comprising:
i. reduced or eliminated surface expression of endogenous T cell receptor alpha chain by a genetic modification in the T cell receptoralpha chain gene,
ii. reduced or eliminated surface expression of HLA-A relative to an unmodified T cell by a genetic modification in the HLA-A gene,
iii. reduced or eliminated surface expression of HLA class II by a genetic modification in the CIITA gene, and
iv. a switchable chimeric antigen cell surface receptorthat comprises:
• a tag-binding domain or a tag,
• an extracellular hinge and a transmembrane domain, and
• a signal transduction domain.

2. The engineered human T cell according to claim 1, wherein the switchable chimeric antigen cell surface receptor is a reversed universal chimeric antigen cell surface receptor comprising a tag, an extracellular hinge and a transmembrane domain, and signal transduction domain.

3. The engineered human T cell according to claim 1 or 2, wherein the tag is a peptide epitope tag.

4. The engineered human T cell according to claim 3, wherein the peptide epitope tag is a myc-tag, a His-tag, a short linear peptide sequence from yeast transcription factor GCN4, a leucine zipper sequence or a short linear peptide sequence from a human nuclear protein, preferably from the human La protein.

5. The engineered human T cell according to claim 1, wherein tag-binding domain is an antibody, antibody fragment, a protein or a peptide.

6. The engineered human T cell according to claim 1 or 2, wherein the tag-binding domain is an antibody or an antibody fragment, a protein or a peptide binding to a myc-tag, a His-tag, a short linear peptide sequence from yeast transcription factor GCN4, a leucine zipper sequence or a short linear peptide sequence from a human nuclear protein, preferably from the human La protein.

7. The engineered human T cell according to one of the claims 1 to 6, wherein the extracellular hinge and transmembrane domain is selected from hinge and transmembrane domains of human CD28 molecule, CD8a chain NK cell receptors, or parts of the constant region of an antibody, mutants thereof and combinations thereof.

8. The engineered human T cell according to one of the claims 1 to 7, wherein the signal transduction domain is selected from cytoplasmic regions of CD28, CD137 (4-1BB), CD134 (OX40), CD278 (ICOS), DAP10 and CD27, programmed cell death-1 (PD-1), cytotoxic T-lymphocyte antigen 4 (CTLA-4), cytoplasmic regions of CD3 chains, DAP12, CD122 (interleukin-2 receptor β), CD132 (interleukin-2 receptor γ), CD127 (interleukin-7 receptor α), CD360 (interleukin-21 receptor), activating Fc receptors, mutants thereof and combinations thereof.

9. The engineered human T cell according to one of the claims 1 to 8, wherein the T cell is homozygous for HLA-B and homozygous for HLA-C genotypes.

10. A pharmaceutical composition comprising the engineered human T cell according to one of the claims 1 to 9.

11. The pharmaceutical composition according to claim 10 for use in a method for stimulating a chimeric antigen receptor-mediated immune response in a mammal, preferably for use in the treatment of cancer, infectious disease or autoimmune disease.

12. A kit comprising
a) an engineered human T cell, comprising:
i. reduced or eliminated surface expression of endogenous T cell receptor alpha chain by a genetic modification in the T cell receptoralpha chain gene,
ii. reduced or eliminated surface expression of HLA-A relative to an unmodified T cell by a genetic modification in the HLA-A gene,
iii. reduced or eliminated surface expression of HLA class II by a genetic modification in the CIITA gene, and
iv. a switchable chimeric antigen cell surface receptor that comprises:
• a tag-binding domain or a tag,
• an extracellular hinge and a transmembrane domain, and
• a signal transduction domain, and
b) a targeting module comprising a tag-binding domain or a tag and at least one target cell-binding domain or a nucleic acid, vector or cell encoding the targeting module,
wherein the tag-binding domain of the targeting module binds to the tag of the switchable chimeric antigen receptor or the tag of the targeting module binds to the tag-binding domain of the switchable chimeric antigen receptor.

13. The kit according to claim 12, wherein the at least one target cell-binding domain is an antibody, antibody fragment, a protein, a peptide or a low molecular weight organic ligand that binds to surface antigens selected from the group comprising CD2, CD3, CD4, CD5, CD7, CD8, CD10, CD15, CD19, CD20, CD22, CD23, CD25, CD30, CD33, CD38, CD44, CD44v6 CD52, CD66a, CD66b, CD66c, CD66d, CD66e, CD66f, CD90, CD99, CD123, CD133, CD135, CD150 CD181, CD182, CD184, CD223, CD229, CD269, CD273, CD274, CD276, CD279, CD319, CD366 and CD371, cytokine receptors, CXCR4, c-Met, mesothelin, members of the epidermal growth factor receptor family and mutants thereof, members of the tumor necrosis factor receptor superfamily, claudins, ephrins, ephrin receptors, fucosyl transferases, prostate specific antigens, embryonic antigens, members of the vascular endothelia growth factor family, EpCAM, AFP, members of the intercellular adhesion molecule family, C-type lectins, integrins, members of the mucin protein family, FSHR, HMW-MAA, FBP, folate receptors, somatostatin receptors, ligands of the NKG2D receptor, members of the epithelia glycoprotein family, diasialogangliosides, glypicans, G protein-coupled receptors, human papillomavirus proteins, cancer-testis antigens, fibroblast activation proteins, members of the carbonic anhydrase family, members of the carbohydrate antigen family, Notch ligands, MCSP, glycoprotein A33, guanylate cyclase 2C and tumor-specific glycans.

14. The kit according to claim 12 or 13, wherein the at least one target cell-binding domain is an antibody, antibody fragment, a protein, a peptide or a low molecular weight organic ligand that binds to CD123.

15. The kit according to one of the claims 12 to 14, wherein the kit further comprises at least one further targeting module or at least one further nucleic acid, vector or cell encoding a further targeting module,
wherein the at least one further targeting module comprises at least one target cell-binding domain and a tag-binding domain or a tag,
wherein the at least one target cell-binding domain is an antibody, antibody fragment, a protein, a peptide or a low molecular weight organic ligand that binds to surface antigens selected from the group comprising CD2, CD3, CD4, CD5, CD7, CD8, CD10, CD15, CD19, CD20, CD22, CD23, CD25, CD30, CD33, CD38, CD44, CD44v6 CD52, CD66a, CD66b, CD66c, CD66d, CD66e, CD66f, CD90, CD99, CD133, CD135, CD150 CD181, CD182, CD184, CD223, CD229, CD269, CD273, CD274, CD276, CD279, CD319, CD366 and CD371, cytokine receptors, CXCR4, c-Met, mesothelin, members of the epidermal growth factor receptor family and mutants thereof, members of the tumor necrosis factor receptor superfamily, claudins, ephrins, ephrin receptors, fucosyl transferases, prostate specific antigens, embryonic antigens, members of the vascular endothelia growth factor family, EpCAM, AFP, members of the intercellular adhesion molecule family, C-type lectins, integrins, members of the mucin protein family, FSHR, HMW-MAA, FBP, folate receptors, somatostatin receptors, ligands of the NKG2D receptor, members of the epithelia glycoprotein family, diasialogangliosides, glypicans, G protein-coupled receptors, human papillomavirus proteins, cancer-testis antigens, fibroblast activation proteins, members of the carbonic anhydrase family, members of the carbohydrate antigen family, Notch ligands, MCSP, glycoprotein A33, guanylate cyclase 2C and tumor-specific glycans.

16. The kit according to one of the claims 12 to 15, wherein the engineered human T cell and/or the targeting module are in the form of a pharmaceutical composition.

17. The kit according to one of the claims 12 to 16 for use in a method for stimulating a chimeric antigen receptor-mediated immune response in a mammal, preferably for use in the treatment of cancer, infectious disease or autoimmune disease.

18. A kit comprising
a) a CRISPR system comprising
• a nucleic acid that causes downregulation of gene expression of an endogenous T cell receptor alpha chain,
• a nucleic acid that causes downregulation of gene expression of HLA-A,
• a nucleic acid that causes downregulation of gene expression of CIITA, and
b) a nucleic acid or a vector comprising a nucleic acid encoding a switchable chimeric antigen cell surface receptor that comprises:
- a tag-binding domain or a tag,
- an extracellular hinge and a transmembrane domain, and
- a signal transduction domain, and
c) a targeting module comprising a tag-binding domain or a tag and at least one target cell-binding domain or a nucleic acid, vector or cell encoding the targeting module,
wherein the tag-binding domain of the targeting module binds to the tag of the switchable chimeric antigen receptor or the tag of the targeting module binds to the tag-binding domain of the switchable chimeric antigen receptor.

19. The kit according to claim 18, wherein the CRISPR system comprises an endonuclease and lipid nanoparticles comprising CRISPR-associated protein 9 mRNA and a target site-specific single guide RNA.

20. The kit according to claim 19, wherein the target site-specific single guide RNA that causes downregulation of gene expression of an endogenous T cell receptor alpha chain comprises a nucleotide sequence encoded by SEQ ID NO. 1.

21. The kit according to claim 19 or 20, wherein the target site-specific single guide RNA that causes downregulation of gene expression of HLA-A comprises a nucleotide sequence encoded by SEQ ID NO. 2.

22. The kit according to one of the claims 19 to 21, wherein the target site-specific single guide RNA that causes downregulation of gene expression of CIITA comprises a nucleotide sequence encoded by SEQ ID NO. 3.

23. A method for generating an engineered human T cell expressing a switchable chimeric antigen receptor, the method comprising:
(1) providing a human T cell,
(2) introducing into a human T cell a CRISPR system comprising a nucleic acid that causes downregulation of gene expression of an endogenous T cell receptoralpha chain in the T cell by transfection,
(3) introducing into the human T cell a CRISPR system comprising a nucleic acid that causes downregulation of gene expression of HLA-A in the T cell by transfection, and
(4) introducing into the human T cell a CRISPR system comprising a nucleic acid that causes downregulation of gene expression of CIITA in the T cell by transfection, thereby generating the CRISPR-modified T cell, and
(5) introducing into to the human T cell a nucleic acid or a vector comprising a nucleic acid encoding a switchable chimeric antigen cell surface receptor that comprises:
• a tag-binding domain or a tag,
• an extracellular hinge and a transmembrane domain, and
• a signal transduction domain,
by transduction.

24. The method for generating an engineered human T cell expressing a switchable chimeric antigen receptor according to claim 23, wherein the T cell is homozygous for HLA-B and homozygous for HLA-C.

25. The method for generating an engineered human T cell expressing a switchable chimeric antigen receptor according to claim 24, wherein the method further comprises matching the engineered human T cell for the HLA-B and HLA-C alleles to a patient.

26. The method for generating an engineered human T cell expressing a switchable chimeric antigen receptor according to one of the claims 23 to 25, wherein the CRISPR system comprises a lipid nucleic acid assembly composition comprising an endonuclease or an mRNA encoding an endonuclease and a target site-specific single guide RNA.

27. The method for generating an engineered human T cell expressing a switchable chimeric antigen receptor according to one of the claims 26, wherein the endonuclease is CRISPR-associated enzyme 9.

28. The method for generating an engineered human T cell expressing a switchable chimeric antigen receptor according to claim 26 or 27, wherein the target site-specific single guide RNA in step (2) comprises a nucleotide sequence according to SEQ ID NO. 1.

29. The method for generating an engineered human T cell expressing a switchable chimeric antigen receptor according to one of the claims 26 to 28, wherein the target site-specific single guide RNA in step (3) comprises a nucleotide sequence according to SEQ ID NO. 2.

30. The method for generating an engineered human T cell expressing a switchable chimeric antigen receptor according to one of the claims 26 to 29, wherein the target site-specific single guide RNA in step (4) comprises a nucleotide sequence according to SEQ ID NO. 3.

31. The method for generating an engineered human T cell expressing a switchable chimeric antigen receptor according to one of the claims 26 to 30, wherein the lipid nucleic acid assembly composition comprises lipid nanoparticles.

32. The method for generating an engineered human T cell expressing a switchable chimeric antigen receptor according to claim 31, wherein the lipid nanoparticles are a mixture of cationic and/or ionizable lipids and helper lipids.

33. The method for generating an engineered human T cell expressing a switchable chimeric antigen receptor according to claims 31 or 32, wherein the lipid nanoparticles are a mixture of an ionizable lipid, a helper lipid and PEG-DMG.

34. The method for generating an engineered human T cell expressing a switchable chimeric antigen receptor according to one of the claims 23 to 33, wherein the introduction of a nucleic acid or a vector comprising a nucleic acid encoding a switchable chimeric antigen cell surface receptor according to step (5) is carried out by adding retroviral, lentiviral, or adenoviral vector particles.
